# FASCICULE DE BREVET EUROPEEN

(11) **EP 3 768 210 B1**
(45) Date de publication et mention de la délivrance du brevet: **16.10.2024**
(21) Numéro de dépôt: 19718434.4
(22) Date de dépôt: 20.03.2019
(51) Int. Cl.: A61F 13/62, A61F 13/15, B32B 5/02, B32B 7/12, B32B 27/12, B32B 27/30, B32B 37/15, B32B 3/30

(54) **ENSEMBLE LAMINE, COUCHE CULOTTE COMPRENANT UN TEL ENSEMBLE ET PROCEDE DE FABRICATION D'UN TEL ENSEMBLE**
LAMINIERTE ANORDNUNG, WINDEL MIT SOLCH EINER ANORDNUNG UND VERFAHREN ZUR HERSTELLUNG EINER SOLCHEN ANORDNUNG
LAMINATED ASSEMBLY, NAPPY COMPRISING SUCH AN ASSEMBLY AND METHOD FOR MANUFACTURING SUCH AN ASSEMBLY

(30) Priorité: 21.03.2018 FR 1852405
(43) Date de publication de la demande: 27.01.2021
(73) Titulaire: Aplix, 44850 Le Cellier (FR)
(72) Inventeur: LINOT, Pierre-Yves François Jean, 44850 LE CELLIER (FR)
(74) Mandataire: Cabinet Beau de Loménie
(86) Numéro de dépôt international: PCT/FR2019/050638
(87) Numéro de publication internationale: WO 2019/180381

(56) Documents cités:
- EP-A2- 1 311 372
- WO-A1-2014/190042
- WO-A1-2017/187097
- FR-A1- 2 943 356
- US-A1- 2013 149 488

## Description

### Domaine technologique

Le présent exposé concerne des ensembles laminés pouvant être utilisés dans le domaine de l'hygiène, en particulier des articles absorbants, notamment pour la fabrication d'oreilles élastiques pour couches culottes.

### Arrière-plan technologique

Une couche culotte jetable est généralement composée d'une partie centrale absorbante comprenant à chaque extrémité une partie avant formant ceinture munie de deux oreilles avant et une partie arrière formant ceinture munie de deux oreilles arrière permettant de fixer la couche culotte sur la personne qui porte la couche culotte jetable. Chaque oreille arrière est généralement munie d'un dispositif de retenue, par exemple à crochets, qui coopère avec une zone d'application disposée sur la ceinture avant. Cette zone d'application est communément appelée dans le domaine de l'hygiène « bande confort » ou identifiée par l'expression en anglais « landing zone ».

Toutefois, il arrive que la couche culotte puisse s'affaisser au niveau des oreilles et/ou que l'oreille avant se déplace par rapport à l'oreille arrière sous l'effet des mouvements du porteur de la couche culotte. Cet affaissement et/ou ce déplacement peuvent entraîner un inconfort pour le porteur de la couche culotte et/ou des fuites non désirées. WO 2014/190042 A1 et US 2013/149488 A1 décrivent des articles absorbants avec une bande anti-glissement. WO 2017/187097 A1 décrit un système de fermeture utilisable dans un article absorbant.

### Présentation

Le présent exposé vise à remédier au moins en partie à ces inconvénients.

A cet effet, le présent exposé concerne un article absorbant, tel qu'une couche culotte jetable comprenant un ensemble laminé comprenant une couche de support et une bande anti-glissement, la bande anti-glissement comprenant un matériau élastomère, la couche de support et la bande anti-glissement étant laminées ensemble, la bande anti-glissement comprenant une base et une pluralité d'éléments en saillie s'étendant de la base et la pluralité d'élément en saillie faisant saillie d'une face de l'ensemble laminé.

Grâce à la bande anti-glissement, et en particulier les éléments en saillie, lorsque l'ensemble laminé est en contact avec une autre surface, le déplacement de cette autre surface par rapport à la bande anti-glissement est réduit. Toutefois, les éléments en saillie ne permettent pas l'assemblage et l'accrochage de la bande anti-glissement avec cette autre surface. Par exemple, la bande anti-glissement est telle qu'il n'est pas possible de suspendre un poids de 1 kg (kilogramme) pendant une durée de 10 secondes. Dans certains cas, la bande anti-glissement a une force de pelage à 180° qui est inférieure ou égale à 0,02 N, dans certain cas égale à 0 N.

On entend par direction MD la direction de déplacement de la bande anti-glissement dans la machine lors de la fabrication de la bande anti-glissement, conformément au sigle anglais pour « Machine Direction », et par direction CD, conformément au sigle anglais pour « Cross Direction », la direction perpendiculaire à la direction MD.

La méthode « Pelage 180° » est une méthode qui permet de mesurer la force de pelage, c'est-à-dire la force pour séparer l'ensemble laminé et la zone d'application. Cette méthode est décrite ci-dessous.

Conditionnement des échantillons - Les échantillons à tester sont conditionnés pendant 2 h (heure) à 23°C+/-2°C avec une humidité relative de 50%+/-5%.

Préparation de la bande anti-glissement - La bande anti-glissement est généralement utilisée dans la direction MD. La bande anti-glissement se présente généralement sous forme d'un ruban dont la longueur est dans la direction MD. Une partie du ruban selon la direction MD est collée sur un papier de 80 g/cm² et un rouleau de 2 kg (kilogramme) est appliqué ou passé en rotation sur la bande anti-glissement dans un sens et puis dans l'autre (aller-retour) sur toute la longueur de la partie du ruban à une vitesse d'environ 700 mm/min (millimètre par minute). Le papier et la bande anti-glissement sont découpés à l'aide d'une paire de ciseaux en bandelettes de 25,4 mm (millimètre) de largeur dans la direction MD. Chaque bandelette de papier présente une longueur de 210 mm et la bande anti-glissement est disposée au centre de cette bandelette.

Préparation de la zone d'application - L'échantillon de la zone d'application présente une largeur de 50 mm dans la direction MD et la longueur est au maximum de 200 mm et l'échantillon est coupé en deux selon la longueur.

Assemblage - La bandelette est disposée sur l'échantillon de la zone d'application de sorte que la bande anti-glissement soit centrée sur l'échantillon de la zone d'application. Le rouleau de 2 kg (kilogramme) est appliqué ou passé en rotation sur la bandelette dans un sens et puis dans l'autre (aller-retour) sur toute la longueur de la bandelette à une vitesse d'environ 700 mm/min. L'échantillon de la zone d'application est disposé dans une pince d'une potence, le côté coupé étant dans la pince et un poids de 1 kg est suspendu à la partie inférieure de la bandelette pendant 10 s (seconde). Le poids est ensuite retiré. Cette étape permet de s'assurer de l'assemblage de la bande anti-glissement et de l'échantillon de la zone d'application.

Mesure - L'ensemble est ensuite disposé dans une machine d'essai en traction comprenant une cellule de mesure de 100 N (newton). La bandelette est insérée dans la mâchoire supérieure (mobile). On met la lecture de la cellule de mesure de force à zéro. On insère l'échantillon de la zone d'application dans la mâchoire inférieure (fixe) et on crée une légère tension. La force doit être comprise entre 0,02 N et 0,05 N. Lors de la mise en place, les mâchoires sont écartées l'une de l'autre de 50 mm. L'ensemble est centré entre les deux mâchoires. Le test est réalisé à déplacement constant à une vitesse de 305 mm/min et la course d'essai est de 50 mm. Cette course d'essai est adaptée en fonction de la largeur du dispositif de retenue à tester.

La bande anti-glissement étant réalisée dans un matériau élastomère, la bande anti-glissement est élastique. L'élasticité de la bande anti-glissement confère de l'élasticité à l'ensemble laminé. Ainsi, lorsque l'on utilise l'ensemble laminé, on peut étirer l'ensemble laminé, la bande anti-glissement étant elle-même étirée. L'étirement de l'ensemble laminé permet d'exercer une pression, lors par exemple de l'attache d'une couche culotte jetable sur un porteur, qui est supérieure à la pression qui serait exercée par l'ensemble laminé s'il n'était pas étiré. Cette pression supérieure permet également de réduire le risque que la surface en contact avec la bande anti-glissement bouge et/ou se déplace par rapport à la bande anti-glissement.

La bande anti-glissement étant réalisée en matériau élastomère, le toucher est doux pour la personne qui porte la couche culotte ainsi que pour la personne qui manipule la couche culotte et minimise le risque de sensibilisation cutanée.

Comme exemple non limitatif de matériau élastomère, on peut citer : les copolymères styrène/isoprène (SI), styrène/isoprène/styrène (SIS), styrène/butadiène/styrène (SBS), styrène-éthylène/butylène-styrène (SEBS), styrène-éthylène/propylène-styrène (SEPS) ou SIBS. Des mélanges de ces élastomères les uns avec les autres ou avec des non élastomères modifiant certaines caractéristiques autres que l'élasticité peuvent également être pris en compte. Par exemple jusqu'à 50% en poids (ou en masse) mais, de préférence, moins de 30% en poids (ou en masse) de polymère peuvent être ajoutés afin de modifier certaines caractéristiques des matériaux de base (élasticité, tenue à la chaleur, processabilité, tenue aux UV, colorant,...), tels que des polyvinyles styrène, des polystyrènes ou des poly a-méthyl-styrène, polyesters époxy, polyoléfines, par exemple des polyéthylènes ou certains acétates d'éthylène/vinyle, de préférence ceux de poids moléculaire élevé (masse molaire plus élevée).

Le matériau élastomère peut être, notamment, un styrène-isoprène-styrène, disponible par exemple auprès de la Société Kraton Polymers, sous la dénomination KRATON D (Marque déposée), ou de la société DEXCO POLYMERS LP sous la dénomination VECTOR SBC 4211 (Marque déposée). On peut également utiliser les matériaux TPE (ThermoPlastic Elastomère), en particulier un élastomère thermoplastique de polyuréthane, notamment le PELLETHANE (Marque déposée) 2102-75A de la Société The Dow Chemical Company. On peut utiliser également un styrène-butadiène-styrène, notamment le KRATON D-2122 (Marque déposée) de la société Kraton Polymers, ou le VECTOR SBC 4461 (Marque déposée) de la société Dexco Polymers LP. On peut aussi utiliser un styrène-éthylène/butylène, notamment le KRATON G-2832 (Marque déposée) de la société Kraton Polymers, ou un copolymère séquence styrène-éthylène-butylène-styrène (SEBS), notamment le KRATON (Marque déposée) G2703.

La liste sans être exhaustive, peut être complétée par l'utilisation de l'ensemble des polymères polyisoprènes hydrogénés tels que le styrène-éthylène-propylène-styrène (SEPS), styrène-éthylène-propylène-styrène-éthylène-éthylène-propylène (SEPSEP), polymères de polybutadiène hydrogénés tels que styrène-éthylène-butylène-styrène (SEBS), styrène-éthylène-butylène-styrène-éthylène-éthylène-butylène-éthylène-butylène-butylène (SEBSEB), styrène-butadiène-styrène (SBS), styrène-isoprène-styrène (SIS), styrène-isoprène-butadiène-styrène (SIBS), polymère polyisoprène/butadiène hydrogéné tel que styrène-éthylène-éthylène-éthylène-épropylène-styrène (SEEPS), et des polymères tribloc vinyle-polyisoprène hydrogéné/polyisoprène hydrogéné/polyisoprène/polystyrène, tels que HYBRAR 7311, disponibles dans le commerce (Kuraray America, Inc., Houston, Tex.), et leurs combinaisons.

Les configurations de blocs polymères tels que diblock, triblock, multiblock, multiblock, star et radial sont également envisagées dans cette divulgation. Dans certains cas, des copolymères séquencés de poids moléculaire (ou masse molaire) plus élevé peuvent être souhaitables. Les copolymères séquencés sont disponibles auprès de Kraton Polymers U.S. LLC de Houston, Tex. sous les désignations, par exemple, Kraton MD6716, Kraton D1102, Kraton SIBS D1102, Kraton D1184, Kraton FG1901 et Kraton FG1924, et Septon Company of America, Pasadena, Tex. sous les dénominations Septon 8007, Septon V9827 et Septon 9618. Dynasol d'Espagne est un autre fournisseur potentiel de ces polymères. En particulier, le polymère tribloc Kraton MD6716 SEBS est particulièrement adapté à la présente divulgation.

On peut également utiliser un copolymère d'acrylate d'isooctyle et d'acide acrylique suivant des rapports de monomère de 90/10, quie st un thermoplastique avec réticulation physique en absence d'un réticulant. On peut également utiliser un copolymère séquence polyamide polyester PEBAX (Marque déposée) 2533 de la société Arkema.

D'autres matériaux possibles sont des polymères polyoléfines, principalement des copolymères d'éthylène et/ou propylène, ayant des caractéristiques des élastomères, notamment issus de la catalyse métallocène, tel le VISTAMAXX VM-1120 (Marque déposée), disponible auprès de la société Exxon Mobil Chemical ou encore des polymères chargés avec du caoutchouc, comme par exemple le Santoprène chargé avec de l'EPDM.

Des exemples d'élastomères thermoplastiques à base de polyoléfine utilisables dans les couches de film élastomère comprennent, entre autres, une polyoléfine cristalline, par exemple, un homopolymère ou un copolymère d'une alpha-oléfine ayant 1 à 20 atomes de carbone, et comprenant 1 à 12 atomes de carbone.

Les homopolymères et les copolymères décrits ci-dessous sont des exemples de polyoléfines cristallines.
(1) Homopolymère d'éthylène L'homopolymère d'éthylène peut être préparé par l'un quelconque des procédés à basse pression et à haute pression.
(2) Copolymères d'éthylène et pas plus de 10 % en moles d'alpha-oléfines autres que l'éthylène ou de monomères vinyliques tels que l'acétate de vinyle et l'acrylate d'éthyle ; par exemple, le copolymère d'éthylène octène, disponible sous les marques Engage 8407 ou Engage 8842 (Dow Chemical, Houston, Tex.).
(3) Homopolymère de propylène ; les exemples comprennent le copolymère à impact de polypropylène PP7035E4 et le copolymère aléatoire de polypropylène PP9574E6 (Exxon Mobil, Houston, Tex.).
(4) Copolymères aléatoires de propylène et pas plus de 10 % en moles d'alpha-oléfines - oléfines autres que le propylène.
(5) Copolymères séquencés de propylène et pas plus de 30 % en moles de alpha-oléfines autres que le propylène.
(6) Homopolymère de butène-1-butène.
(7) Copolymères aléatoires de 1-butène et pas plus de 10 % en moles d'alpha-oléfines - oléfines autres que 1-butène.
8) Homopolymère de 4-méthyl-1-pentène homopolymère de 4-méthyl-1-pentène.
(9) Copolymères aléatoires de 4-méthyl-1-pentène et pas plus de 20 % en moles d'alpha-oléfines autres que le 4-méthyl-1-pentène.

Les alpha-oléfines comprennent, par exemple, l'éthylène, le propylène, le butène-1, le 4-méthyl-1-pentène, le 1-hexène et le 1-octène.

Parmi les élastomères thermoplastiques à base de polyoléfines disponibles dans le commerce et destinés à être utilisés dans les couches de film élastomère, on peut citer VISTAMAXX.TM. (élastomère à base de propylène, disponible chez ExxonMobil Chemical, Houston, Tex.), INFUSE.TM. (copolymères séquencés d'oléfines, disponibles auprès de Dow Chemical Company, Midland, Michigan), VERSIFY.TM. (copolymères propylène-éthylène) tels que VERSIFY.TM. 4200 et VERSIFY.TM. 4300 (Dow Chemical Company, Midland, Michigan), ENGAGE.TM. (copolymère d'éthylène octane, disponible auprès de Dow Chemical, Houston, Tex.) et NOTIO 0040 et NOTIO 3560 (disponible auprès de Mitsui Chemical (USA), New York, N.Y.), Adflex X100 G (disponible auprès de Lyondellbasell).

Dans une réalisation particulièrement adaptée, l'élastomère thermoplastique à base de polyoléfine est VISTAMAXX.TM. 6102FL ou VISTAMAXX 7050 FLX (disponible chez ExxonMobil Chemical, Houston, Tex.). La référence « TM » pour les noms de marques enregistrées correspond à « Trade Mark ».

Dans un autre cas, l'élastomère thermoplastique peut être un élastomère ester/éther thermoplastique ou des polyuréthanes thermoplastiques.

On entend par matériau élastomère, un matériau qui peut être étiré sans se rompre sous l'effet d'une force d'étirement exercée selon une direction donnée et pouvant reprendre sensiblement sa forme et ses dimensions initiales après relâchement de cette force d'étirement. Il s'agit par exemple d'un film qui conserve une déformation résiduelle ou rémanence après élongation et relâchement (déformation résiduelle aussi appelée « permanent set » ou « SET ») inférieure ou égale à 30%, de préférence inférieure ou égale à 20%, encore plus de préférence inférieure ou égale à 10%, de sa dimension initiale (avant élongation) pour un allongement de 100% de sa dimension initiale, à température ambiante (23°C - degré Celsius). Le matériau élastomère peut être un matériau thermoplastique élastomère, en particulier un matériau thermoplastique élastomère réticulé physiquement, tels que ceux décrits dans la présente divulgation, ou un matériau thermoplastique élastomère réticulé chimiquement.

Dans certains modes de réalisation, la bande anti-glissement présente une déformation résiduelle inférieure ou égale à 30%, de préférence inférieure ou égale à 20%, encore plus de préférence inférieure ou égale à 15%, encore plus de préférence inférieure ou égale à 10% de sa dimension initiale (avant élongation) pour un allongement de 100% de sa dimension initiale, à température ambiante (23°C).

Dans certains modes de réalisation, l'ensemble laminé présente une déformation résiduelle inférieure ou égale à 30%, de préférence inférieure ou égale à 20%, encore plus de préférence inférieure ou égale à 15%, encore plus de préférence inférieure ou égale à 10% de sa dimension initiale (avant élongation) pour un allongement de 100% de sa dimension initiale, à température ambiante (23°C).

Dans certains modes de réalisation, la couche de support comprend une nappe de non-tissé.

On entend par nappe de non-tissé un produit obtenu à l'issue de la formation d'un voile de fibres et/ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de ruban ou voile de fibres et/ou filaments qui sont entrelacés d'une manière non uniforme, irrégulière ou au hasard. Un non-tissé peut être réalisé à partir de différents matériaux synthétiques et/ou naturels. Les matériaux naturels à titre d'exemple sont des fibres de cellulose, telles que le coton, la jute, le lin et analogue et peuvent également inclure des fibres de cellulose retraitées, telles que la rayonne ou la viscose. Les fibres naturelles pour une nappe de non-tissé peuvent être préparées en utilisant divers procédés tels que le cardage. Des matériaux synthétiques à titre d'exemple comportent, mais sans s'y limiter, des polymères plastiques synthétiques, qui sont connus pour former des fibres qui incluent, sans s'y limiter, les polyoléfines, par exemple le polyéthylène, polypropylène, polybutylène et analogue ; le polyamide, par exemple le polyamide 6, polyamide 6.6, polyamide 10, polyamide 12 et analogue ; des polyesters, par exemple des polyéthylènes téraphthalates, des polybutylènes téréphtalates, des acides polylactiques et analogues, des polycarbonates, des polystyrènes, des élastomères thermoplastiques, des vinyles polymères, des polyuréthanes et des mélanges et des co-polymères de ces derniers.

Dans certains modes de réalisation, la couche de support peut avoir une structure de couche unique ou une structure à couches multiples. La couche de support peut également être réunie à un autre matériau pour former un stratifié. A titre d'exemple, le non-tissé peut être un non-tissé du type Spunbond, Spunmelt, cardé thermolié, et la couche de support peut être un SMS, SMMS, SS, SSS, SSMMS, SSMMMS, Air through ou autre. Ces exemples sont donnés à titre non limitatif.

Une nappe de non-tissé est par exemple formée d'un voile de fibres et/ou filaments issue de la technologie Dry-laid (voie sèche), Wet-laid (voie humide), ou Spun-laid (voie fondue/extrudée) et consolidés par liaison mécanique, thermique, chimique et/ou adhésive.

La nappe de non-tissé peut être un non-tissé cardé calandré.

Le non-tissé cardé calandré est un non-tissé comprenant un voile de fibres présentant des points de consolidations de la nappe répartis de manière sensiblement homogène sur tout le voile par une consolidation thermique. La consolidation assure une certaine cohésion des fibres permettant leur manipulation et leur transport, notamment leur enroulement sous forme de bobine et leur déroulement. L'activation de la nappe de non-tissé cardé calandré permet d'allonger et/ou de casser les fibres de la nappe de non-tissé et/ou de déformer les points de consolidation de la nappe. On augmente ainsi la capacité d'élongation de la nappe de non-tissé.

Les fibres de la nappe de non-tissé cardé calandré sont comprises entre 1 et 8 dTex (déciTex), de préférence entre 1,3 et 6,7 dTex, encore plus de préférence entre 1,6 et 5,5 dTex.

Le Tex est l'unité SI de finesse des fibres textiles. Elle exprime le poids en gramme de 1 000 m (mètre) de longueur des fibres.

Dans certains modes de réalisation, la couche de support comprend une nappe de non-tissé formant un voile d'acquisition, en particulier un voile d'acquisition pour article absorbant.

Dans certains modes de réalisation, la couche de support comprend un film thermoplastique.

On entend par film thermoplastique un film en matériau thermoplastique qui peut être un matériau élastique ou un matériau non-élastique.

On entend par film thermoplastique en matériau élastique, un film qui peut être étiré sans se rompre sous l'effet d'une force d'étirement exercée selon une direction donnée et pouvant reprendre sensiblement sa forme et ses dimensions initiales après relâchement de cette force d'étirement. Il s'agit par exemple d'un film qui conserve une déformation résiduelle ou rémanence après élongation et relâchement (déformation résiduelle aussi appelée « permanent set » ou « SET ») inférieure ou égale à 30%, de préférence inférieure ou égale à 20%, encore plus de préférence inférieure ou égale à 10%, de sa dimension initiale (avant élongation) pour un allongement de 100% de sa dimension initiale, à température ambiante (23°C).

On entend par film thermoplastique en matériau non-élastique un film qui n'entre pas dans la définition d'un film thermoplastique en matériau élastique.

Lorsque le film thermoplastique est en matériau non-élastique, l'élasticité de l'ensemble laminé est conférée par le matériau élastomère de la bande anti-glissement, par exemple en utilisant un non-tissé avec un grammage inférieur à 30 g/m² (gramme par mètre carré).

Comme exemple non limitatif de matériau thermoplastique, on peut citer une polyoléfine, le polyéthylène, le LLDPE (Linear Low Density PolyEthylène ou polyéthylène linéaire de faible densité), LDPE (Low Density PolyEthylène ou polyéthylène de faible densité), m-PE (Métallocène PolyEthylène), HDPE (High Density PolyEthylène ou polyéthylène de haute densité), EVA (Éthylène Acétate de Vinyle) et PP (PolyPropylène), comprenant une distribution de poids moléculaire (masse molaire) monomodale ou multimodale (par exemple bimodale), en particulier une composition comprenant du LLDPE et d'un plastomère, notamment d'un plastomère base polyéthylène. On pourrait également utiliser du polyamide (PA), de l'acide polyactique (PLA), du polyhydroxyalcanoates (PHA), PVOH, PBS, le polyester, le polychlorure de vinyle (PVC) ou de l'acrylonitrile butadiène styrène (ABS).

Dans certains modes de réalisation, le film thermoplastique est un film élastique.

Comme exemple non limitatif de matériau élastomère pour former le film élastique, on peut citer les mêmes matériaux que les matériaux élastomères cités comme exemples non limitatifs de matériau élastomères pour la réalisation de la bande anti-glissement.

Dans certains modes de réalisation, le film élastique peut être formé par une colle élastique.

Dans certains modes de réalisation, le film élastique peut comprendre plus d'une couche ou être un « skinlayer », c'est-à-dire un film élastique recouvert d'une peau.

Dans certains modes de réalisation, le film élastique peut être formé par une colle élastomère.

Dans certains modes de réalisation, le film de colle élastomère peut être extrudé sur la nappe de non-tissé et ensuite laminé avec cette nappe de non-tissé.

Dans certains modes de réalisation, la couche de support comprend une nappe de non-tissé et le film élastique.

Dans certains modes de réalisation, la base de la bande anti-glissement et le film élastique présentent chacun une largeur, la largeur de la base étant inférieure à la largeur du film élastique, de préférence la largeur de la base étant supérieure ou égale à 10% de la largeur du film élastique et inférieure ou égale à 60% de largeur du film élastique.

Dans certains modes de réalisation, la zone des éléments en saillie de la bande anti-glissement et la base de la bande anti-glissement présentent chacune une largeur, la largeur de la zone des éléments en saillie étant inférieure à la largeur de la base de la bande anti-glissement, de préférence inférieure ou égale à 60% de la largeur de la base, encore plus de préférence inférieure ou égale à 45% de la largeur de la base.

Dans certains modes de réalisation, la couche de support comprend une première nappe de non-tissé, une deuxième nappe de non-tissé et le film élastique, le film élastique étant laminé entre les première et deuxième nappes de non-tissé.

Dans certains modes de réalisation, au repos, dans une zone comprenant les éléments en saillie, la bande anti-glissement présente un coefficient de frottement statique, mesuré selon la norme ASTM D1894, selon la direction MD et/ou la direction CD, supérieur ou égal à 0,1, de préférence supérieur ou égal à 0,5, encore plus de préférence supérieur ou égal à 0,8 et inférieur ou égal à 10, de préférence inférieur ou égal à 5, encore plus de préférence inférieur ou égal à 3.

On entend par « au repos » le fait que l'ensemble laminé n'est pas soumis à des forces externes, telles que des forces d'étirement. En d'autres termes, on entend par « au repos », le fait que l'ensemble laminé est testé tel qu'il est avant la première utilisation par l'usager final, par exemple, dans le domaine des couches culottes, en sortie du conditionnement, c'est-à-dire juste avant de positionner la couche culotte sur une personne. La mesure du coefficient de frottement statique peut être réalisé selon une direction longitudinale et/ou une direction latérale, la direction latérale étant orthogonale à la longitudinale. La mesure du coefficient de frottement statique peut être réalisée selon une direction MD et/ou une direction CD.

On entend par direction MD la direction de déplacement de la bande anti-glissement dans la machine lors de la fabrication de la bande anti-glissement, conformément au sigle anglais pour « Machine Direction », et par direction CD, conformément au sigle anglais pour « Cross Direction », la direction perpendiculaire à la direction MD.

Dans certains modes de réalisation, la bande anti-glissement présente un coefficient de frottement statique, mesuré selon la norme ASTM D1894, lorsque la bande anti-glissement est étirée à 15% de la valeur au repos, compris entre 50% et 150% du coefficient de frottement statique au repos.

La mesure du coefficient de frottement statique peut être réalisée selon une direction longitudinale et/ou une direction latérale, la direction latérale étant orthogonale à la longitudinale. La mesure du coefficient de frottement statique peut être réalisé selon une direction MD et/ou une direction CD.

La bande anti-glissement présente une dimension selon une direction MD qui est plus importante qu'une dimension selon la direction CD.

Dans certains modes de réalisation, la bande anti-glissement présente un grammage par unité de surface supérieur ou égal à 10 g/m² (gramme par mètre carré), de préférence supérieur ou égal à 20 g/m² et inférieur ou égal à 250 g/m², de préférence inférieur ou égal à 200 g/m².

Dans certains modes de réalisation, la couche de support est assemblée par collage.

La colle peut être déposée de manière continue selon une direction longitudinale et de manière discontinue selon une direction latérale. La colle forme ainsi une pluralité de lignes de colle, par exemple continues dans la direction longitudinale. On peut bien entendu adapter la largeur des lignes de colle et leur espacement dans la direction latérale.

Afin de mesurer les caractéristiques propres au matériau élastomère, il est possible de séparer la couche de support du matériau élastomère en utilisant, par exemple, de l'acétone et/ou de l'acétate d'éthyle.

Le film élastique peut être formé par une colle élastique.

Le film de colle élastique peut alors être extrudé sur la nappe de non-tissé et ensuite laminé avec cette nappe de non-tissé. La zone activée peut s'étendre sur toute la longueur de la nappe de non-tissé mesurée dans la direction longitudinale.

Dans certains modes de réalisation, la couche de support est assemblée par soudure ultrason.

Lors de la fabrication d'ensembles laminés, la soudure ultrason est réalisée en faisant passer la couche de support entre deux cylindres, l'un des cylindres étant une sonotrode. Les deux cylindres, dont la sonotrode, appliquent, sur la couche de support, une force perpendiculaire au plan général défini par la couche de support de sorte que lors de la soudure ultrason, la couche de support est laminée.

Dans certains modes de réalisation, la couche de support est assemblée par deux procédés choisis parmi la liste suivante : soudure ultrason, soudures hautes fréquences, colle ou laminage directe (également appelé laminage à chaud).

Dans certains modes de réalisation, au repos, la pluralité d'éléments en saillie présente une densité d'éléments en saillie supérieure ou égale à 3 éléments en saillie par cm², de préférence supérieure ou égale à 10 éléments en saillie par cm² et inférieure ou égale à 400 éléments en saillie par cm², de préférence inférieure ou égale à 300 éléments en saillie par cm².

Dans certains modes de réalisation, au repos, la pluralité d'éléments en saille présente un motif comprenant une répétition d'un motif de bande anti-glissement, le motif de bande anti-glissement s'étendant sur une largeur de la bande anti-glissement.

La bande anti-glissement présente une dimension selon une direction MD qui est plus importante qu'une dimension selon la direction CD.

Dans certains modes de réalisation, une somme des surfaces définies sur la base par les projections orthogonales des éléments en saillie sur la base, est supérieure ou égale à 1% de la surface totale de la base du motif de bande anti-glissement, de préférence supérieure ou égale à 5% et inférieure ou égale à 60% de la surface totale de la base du motif de bande anti-glissement, de préférence inférieure ou égale à 40%, encore plus de préférence inférieure ou égale à 35%.

Dans certains modes de réalisation, la base présente une épaisseur supérieure ou égale à 10 µm, de préférence supérieure ou égale à 15 µm et inférieure ou égale à 200 µm, de préférence inférieure ou égale à 150 µm.

Dans certains modes de réalisation, l'épaisseur de la base est variable.

Dans certains modes de réalisation, les éléments en saillie présentent une largeur variable.

La largeur des éléments en saillie est mesurée dans un plan parallèle au plan XY de la base. La largeur des éléments en saillie est mesurée à l'endroit de l'élément en saillie présentant une largeur maximale.

Dans certains modes de réalisation, les éléments en saillie peuvent comprendre des éléments en saillie présentant des hauteurs différentes et/ou des largeurs différentes mesurées dans un plan parallèle au plan XY.

Dans certains modes de réalisation, les éléments en saillie sont des picots et/ou des plots et/ou des tiges, chaque tige comportant une tête disposée à une extrémité de la tige opposée à la base.

La tête est disposée à une extrémité de l'élément en saillie opposée à la base, en particulier à la face supérieure de la base.

On entend par « picot » une forme dépourvue de tête ou de surplomb et présentant une hauteur maximale supérieure ou égale à une largeur maximale. On entend par « plot » une forme présentant une hauteur maximale inférieure à une largeur maximale. Les picots, plots ou tiges ont une portion présentant une section constante ou une portion présentant une section décroissante, la décroissance étant tournée du côté opposé à la base.

Dans certains modes de réalisation, les éléments en saillie présentent une hauteur, selon une direction perpendiculaire à la base, supérieure ou égale à 0,05 mm (millimètre), de préférence supérieure ou égale à 0,10 mm et inférieure ou égale à 0,80 mm, de préférence inférieure ou égale à 0,50 mm.

Dans certains modes de réalisation, au repos, une largeur de la bande anti-glissement est supérieure ou égale à 5% d'une largeur totale de l'ensemble laminé, de préférence supérieure ou égale à 10% et inférieure ou égale à 45%, de préférence inférieure ou égale à 30%.

Ces valeurs sont appropriées, par exemple, pour des applications pour des couches culottes pour bébé ou enfant ou des couches culottes pour incontinence adulte.

Dans certains modes de réalisation, au repos, une largeur de la bande anti-glissement est comprise entre 25% et 75% d'une largeur totale de l'ensemble laminé.

Ces valeurs sont appropriées, par exemple, pour des applications destinées aux articles absorbants, par exemple à des couches culottes pour bébé ou enfant ou à des couches culottes pour incontinence adulte ou à des articles pour l'hygiène féminine. Ces valeurs peuvent être mesurées une fois que l'article absorbant est assemblé.

Dans certains modes de réalisation, au repos, une largeur de la bande anti-glissement est comprise entre 55% et 100% d'une largeur totale de l'ensemble laminé.

Ces valeurs sont appropriées, par exemple, pour des applications destinées aux articles absorbants, par exemple à des articles pour l'hygiène féminine. Ces valeurs peuvent être mesurées une fois que l'article absorbant est assemblé.

Dans certains modes de réalisation, la couche de support pénètre au moins partiellement dans la base de la bande anti-glissement, par exemple la couche de support est assemblée par lamination directe à la bande anti-glissement.

Dans certains modes de réalisation, la bande anti-glissement est collée sur la couche de support.

Dans certains modes de réalisation, la bande anti-glissement est soudée par soudure ultrason sur la couche du support.

Dans certains modes de réalisation, la bande anti-glissement et le film élastique sont réalisés dans le même matériau élastomère.

Dans certains modes de réalisation, la bande anti-glissement et le film élastique sont réalisés dans des matériaux élastomères différents.

Dans certains modes de réalisation, la nappe de non-tissé est activée avant et/ou après assemblage avec le film élastique.

Dans certains modes de réalisation, la base de la bande anti-glissement comprend deux bords selon une direction longitudinale, l'un desdits bords présente des monts et des vallées, dans lequel un écart maximum entre les monts et les vallées, selon une direction latérale, orthogonale à la direction longitudinale, est inférieur à 1 mm sur une longueur, selon la direction longitudinale, correspondant à trois monts consécutifs.

Dans certains modes de réalisation, les bords présentent, en vue en coupe transversale à la direction longitudinale, une portion de forme arrondie.

Dans certains modes de réalisation, l'écart maximum entre les monts et les vallées, selon une direction transversale à la direction longitudinale et sur une longueur selon la direction longitudinale correspondant à trois monts consécutifs, est compris entre 0,001 mm et 1 mm, plus particulièrement entre 0,001 mm et 0,5 mm, encore plus particulièrement entre 0,001 mm et 0,1 mm.

Dans certains modes de réalisation, les trois monts consécutifs sont sur une distance inférieure à une distance correspondant à 15 pas d'éléments en saillie, de préférence inférieure à une distance de 25 mm.

Dans certains modes de réalisation, la largeur de la base est supérieure ou égale à 1 mm, de préférence supérieure ou égale à 3 mm, encore plus de préférence supérieure ou égale à 5 mm et inférieure ou égale à 500 mm, de préférence inférieure ou égale à 250 mm, encore plus de préférence inférieure ou égale à 100 mm.

Dans certains modes de réalisation, la tige des éléments en saillie présente une symétrie de rotation autour d'un axe perpendiculaire à la face supérieure de la base.

Dans certains modes de réalisation, les éléments en saillie présentent une géométrie asymétrique par rapport à une direction transversale à la direction longitudinale de la base.

Dans certains modes de réalisation, les éléments en saillie présentent une symétrie par rapport à un plan s'étendant selon une direction longitudinale de la base et passant par l'axe de la tige des éléments en saillie.

Dans certains modes de réalisation, la couche de support comprend une première nappe de non-tissé et une deuxième nappe de non-tissé.

Dans certains modes de réalisation, les première et deuxième nappes de non-tissé sont de même nature.

Dans certains modes de réalisation, les première et deuxième nappes de non-tissé sont de nature différente.

Dans certains modes de réalisation, les première et deuxième nappes de non-tissé comprennent chacune des zones non activées 3 et des zones activées préalablement à l'assemblage.

La bande anti-glissement peut être assemblée par collage et/ou par soudure ultrason sur la face supérieure de la couche de support. La bande anti-glissement peut également être assemblée par laminage direct de la bande anti-glissement avant solidification complète de la base de la bande anti-glissement de manière à faire pénétrer au moins partiellement la couche de support dans la base.

Dans le cas où la couche de support est un ensemble de fibres et/ou filaments consolidés thermiquement, la liaison avec la base est également réalisée par pénétration dans la base d'une partie des fibres et/ou filaments de la couche de support.

Dans le cas où la couche de support est une nappe de non-tissé, le démoulage des éléments en saillie est réalisé de manière aisée même avec un non-tissé dont le grammage est inférieur à 80 g/m² (masse de matière en gramme par mètre carré de non-tissé). A titre d'exemple, le grammage du non-tissé peut être compris entre 5 g/m² et 120 g/m², ou encore entre 25 g/m² et 100 g/m², ou encore entre 10 g/m² et 70 g/m².

Ce mode de solidarisation d'une couche de support à une base comprenant des éléments en saillie est notamment avantageux en ce qu'il n'entraine pas une déformation de la base, et permet donc avantageusement de conserver la forme de la base obtenue lors de l'étape d'injection, et notamment de conserver les bords droits pouvant être obtenus via le procédé et l'appareillage décrits ci-après.

Le présent exposé concerne aussi un article absorbant, en particulier une couche culotte jetable, comprenant un ensemble laminé tel que défini précédemment.

Dans certains modes de réalisation, la bande anti-glissement de l'ensemble laminé est agencée sur une oreille élastique de l'article absorbant et/ou sur une oreille non élastique de l'article absorbant, l'oreille non élastique comprenant par exemple un non-tissé.

Dans certains modes de réalisation, la bande anti-glissement de l'ensemble laminé est agencée sur une portion élastique de l'article absorbant, par exemple l'oreille élastique, de telle sorte que les éléments en saillie de la bande anti-glissement s'étendent vers une partie absorbante de l'article absorbant.

Dans certains modes de réalisation, la bande anti-glissement de l'ensemble laminé est agencée sur une portion non-élastique de l'article absorbant, par exemple l'oreille non-élastique, de telle sorte que les éléments en saillie de la bande anti-glissement s'étendent à l'opposé d'une partie absorbante de l'article absorbant.

Le présent exposé concerne également un procédé de fabrication d'un ensemble laminé pour un article absorbant, tel qu'une couche culotte jetable, le procédé comprenant les étapes suivantes :
- formation d'une bande anti-glissement comprenant une base et une pluralité d'éléments en saillie s'étendant de la base par distribution d'un matériau élastomère dans un dispositif de moulage ;
- assemblage d'une couche de support et de la bande anti-glissement par laminage de la couche de support et de la bande anti-glissement.

On entend par direction MD la direction de déplacement de la couche de support dans la machine lors de la fabrication de l'ensemble laminé, conformément au sigle anglais pour « Machine Direction », et par direction CD, conformément au sigle anglais pour « Cross Direction », la direction perpendiculaire à la direction MD.

Dans certains modes de réalisation, le dispositif de moulage comprend une bande de moulage dont l'épaisseur est comprise entre 100 et 500 µm (micromètre).

Dans certains modes de réalisation, l'assemblage de la couche de support et de la bande anti-glissement est réalisé avant solidification complète de la base de la bande anti-glissement de manière à faire pénétrer au moins partiellement la couche de support dans la base.

Dans certains modes de réalisation, la couche de support comprend une nappe de non-tissé et la distribution du matériau élastomère dans le dispositif de moulage est réalisée à travers la nappe de non-tissé.

Dans certains modes de réalisation, l'assemblage de la couche de support et de la bande anti-glissement est réalisé par collage.

Dans certains modes de réalisation, l'assemblage de la couche de support et de la bande anti-glissement est réalisé par soudure ultrason.

Dans certains modes de réalisation, la couche de support comprend un film élastique, le film élastique étant formé par distribution d'un matériau élastomère dans le dispositif de moulage.

Dans certains modes de réalisation, la couche de support comprend un film élastique et au moins un non-tissé, la bande anti-glissement étant assemblée sur la couche de support par la au moins une couche de non-tissé.

Dans certains modes de réalisation, la couche de support comprend une nappe de non-tissé et un film élastique, le film élastique étant réalisé par distribution d'un matériau élastomère dans le dispositif de moulage et l'assemblage de la nappe de non-tissé est réalisé avant solidification complète du film élastique de manière à faire pénétrer au moins partiellement la nappe de non-tissé dans le film élastique.

Dans certains modes de réalisation, le matériau élastomère du film élastique est distribué dans le dispositif de moulage après la distribution du matériau élastomère de la bande anti-glissement.

Dans certains modes de réalisation, la bande anti-glissement et le film élastique sont réalisés dans le même matériau élastomère.

Dans certains modes de réalisation, la couche de support comprend une deuxième nappe de non-tissé, l'assemblage de la deuxième nappe de non-tissé étant réalisé avant solidification complète du film élastique de manière à faire pénétrer au moins partiellement la deuxième nappe de non-tissé dans le film élastique.

Dans certains modes de réalisation, la couche de support comprend une deuxième nappe de non-tissé, l'assemblage de la deuxième nappe de non-tissé étant réalisé par collage et/ou par soudure ultrason de la deuxième nappe de non-tissé sur le film élastique.

Dans certains modes de réalisation, le dispositif de moulage comprend une bande de moulage et des moyens d'entraînement en rotation, la bande anti-glissement étant formée sur la bande de moulage.

Dans certains modes de réalisation, le dispositif de moulage comprend un dispositif de formage des têtes des éléments en saillie comprenant un rouleau d'entrainement et un rouleau de formage.

Dans certains modes de réalisation, le rouleau d'entrainement et le rouleau de formage tournent à des vitesses différentes, de sorte que la tête des éléments en saillie est asymétrique.

Dans certains modes de réalisation, un ratio de la vitesse du rouleau d'entrainement sur la vitesse du rouleau de formage est supérieur ou égal à 0,4, de préférence supérieur ou égal à 0,65 et inférieur ou égal à 1,6, de préférence inférieur ou égal à 1,35.

Dans certains modes de réalisation, un ratio de la vitesse du rouleau d'entrainement sur la vitesse du rouleau de formage est supérieur ou égal à 0,4, de préférence supérieur ou égal à 0,65 et inférieur strictement à 1.

Dans certains modes de réalisation, un ratio de la vitesse du rouleau d'entrainement sur la vitesse du rouleau de formage est égal à 1.

Dans certains modes de réalisation, le rouleau d'entrainement et le rouleau de formage tournent à des vitesses égales, de sorte que la tête des éléments en saillie est symétrique et plate.

### Brève description des dessins

D'autres caractéristiques et avantages de l'objet du présent exposé ressortiront de la description suivante de modes de réalisation, donnés à titre d'exemples non limitatifs, en référence aux figures annexées, sur lesquelles :
- la figure 1 est une vue schématique d'une couche culotte ;
- la figure 2A est une vue schématique en coupe, selon le plan II-II de la figure 1, d'un ensemble laminé selon un premier mode de réalisation ;
- la figure 2B est une vue schématique éclatée en coupe d'une couche de support ;
- les figures 3 à 5 sont des vues schématiques en coupe d'un ensemble laminé selon d'autres modes de réalisation ;
- la figure 6 est une vue schématique en coupe d'une bande anti-glissement ;
- la figure 7 est une vue schématique partielle en coupe d'un ensemble laminé selon un autre mode de réalisation ;
- les figures 8A-8F sont des vues schématiques en coupe et du dessus de différents modes de réalisation de la bande anti-glissement ;
- la figure 9 est une vue schématique partielle du dessus d'une bande de moulage ;
- les figures 10 et 11 sont des vues schématiques en coupe de la bande de moulage de la figure 9 ;
- la figure 12 est une vue schématique agrandie d'un motif de la zone XII de la bande de moulage de la figure 9 ;
- la figure 13 est une vue schématique d'un motif d'éléments d'une bande de moulage selon un autre mode de réalisation ;
- la figure 14 est une vue schématique partielle en coupe selon le plan XIV-XIV de la bande de moulage de la figure 13 ;
- les figures 15-18 sont des vues schématiques de motifs de bandes de moulage selon d'autres modes de réalisation ;
- la figure 19 une vue schématique partielle en coupe selon le plan XIX-XIX de la bande de moulage permettant de former le motif de la figure 18 ;
- la figure 20 est une représentation schématique d'un exemple d'appareillage pour la réalisation d'une bande anti-glissement ;
- les figures 21 à 23 sont des représentations schématiques d'exemples d'appareillages pour la réalisation d'un ensemble laminé ;
- les figures 24A-24C sont des représentations schématiques d'ensembles laminés ;
- la figure 25 est une représentation schématique d'un échantillon de test ;
- la figure 26 est une représentation schématique d'un équipement utilisé pour réaliser la mesure d'allongement à la rupture et/ou la mesure de la rémanence ;
- la figure 27 est un graphique représentant les courbes d'allongement à la rupture d'un ensemble laminé comprenant une bande anti-glissement et d'un ensemble laminé dépourvu d'une telle bande anti-glissement ;
- la figure 28 est une représentation schématique d'un exemple d'appareillage pour la réalisation d'une bande anti-glissement comprenant un dispositif de formage ;
- la figure 29 est une vue de dessus d'une bande anti-glissement illustrant les propriétés des bordures de cette bande.

Sur l'ensemble des figures, les éléments en commun sont repérés par des références numériques identiques.

### Description détaillée

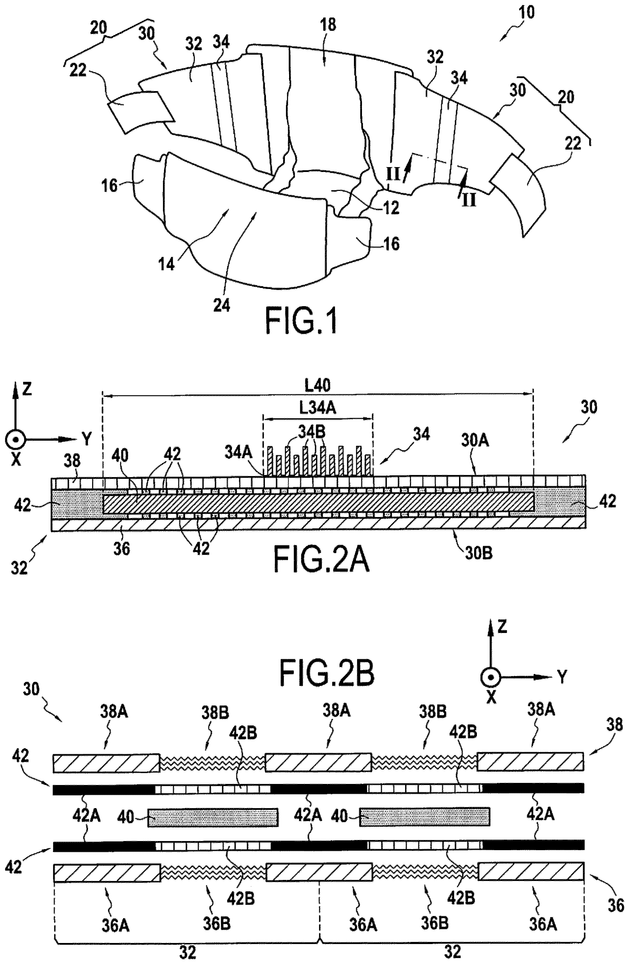

La figure 1 représente de manière très schématique, une couche culotte 10, par exemple une couche culotte jetable. La couche culotte 10 comprend une partie centrale 12 absorbante et une ceinture avant 14 munie de deux oreilles avant 16 et une ceinture arrière 18 munie de deux oreilles arrière 20 permettant de fixer la couche culotte 10 sur la personne qui porte la couche culotte. Chaque oreille arrière 20 est généralement munie d'un dispositif de retenue 22, par exemple à crochets, qui coopère avec une zone d'application 24 disposée sur la ceinture avant 14. Cette zone d'application 24 est communément appelée dans le domaine de l'hygiène « bande confort » ou identifiée par l'expression en anglais « landing zone ».

La figure 2A représente un premier mode de réalisation d'un ensemble laminé 30 pouvant être utilisé pour fabriquer une oreille avant 16 et/ou une oreille arrière 20 pour couche culotte 10.

Par la suite l'expression ensemble laminé désignera aussi bien l'ensemble laminé non découpé que l'ensemble laminé découpé pour former l'oreille avant 16 et/ou l'oreille arrière 20 d'une couche culotte 10.

L'ensemble laminé 30 est représenté sur la figure 2A en vue en coupe selon le plan de coupe II-II de la figure 1.

L'ensemble laminé 30 s'étend selon une direction longitudinale X et une direction latérale Y, orthogonale à la direction longitudinale X. La vue en coupe de la figure 2A est selon le plan de coupe YZ, la direction transversale Z étant orthogonale au plan XY et définissant la direction de l'épaisseur de l'ensemble laminé 30. Les directions XYZ sont dont orthogonales entre elles.

L'ensemble laminé 30 de la figure 2A comprend une couche de support 32 et une bande anti-glissement 34. La couche de support 32 et la bande anti-glissement 34 s'étendent selon la direction longitudinale X et la direction latérale Y. L'ensemble laminé 30 comprend une face supérieure 30A et une face inférieure 30B.

Dans le mode de réalisation de la figure 2A, la couche de support 32 comprend une première nappe de non-tissé 36, une deuxième nappe de non-tissé 38 et un film élastique 40, assemblés par de la colle 42. Le film élastique 40 est assemblé entre la première nappe de non-tissé 36 et à la deuxième nappe de non-tissé 38 par laminage des première et deuxième nappe de non-tissé 36, 38, du film élastique 40 et de la colle 42.

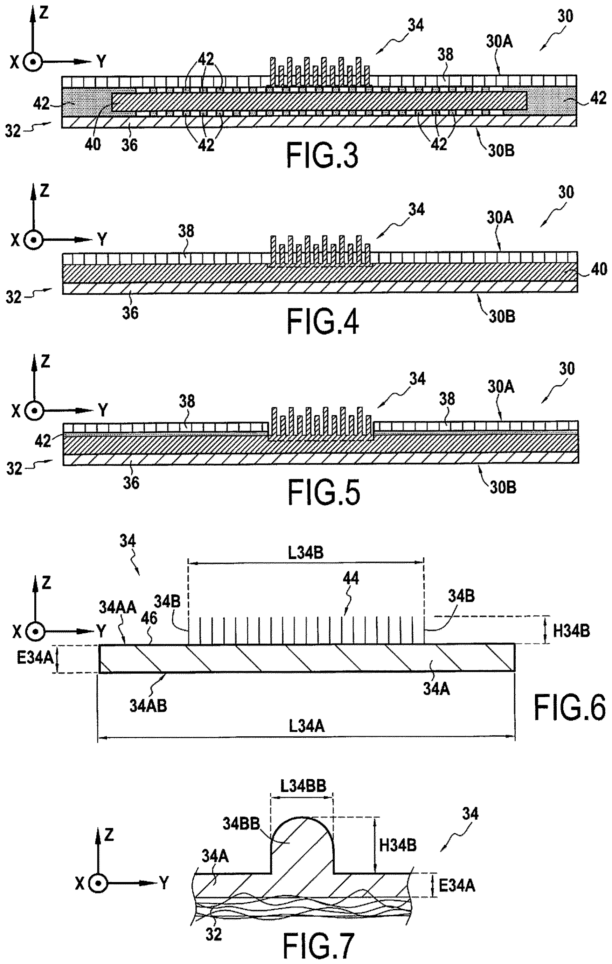

Comme représenté sur la figure 6, la bande anti-glissement 34 comprend une base 34A et une pluralité d'éléments en saillie 34B. La base 34A comporte une face supérieure 34AA et une face inférieure 34AB et les éléments en saillie 34B s'étendent depuis la base 34A, en particulier depuis la face supérieure 34AA de la base 34A. Selon la direction latérale Y, la base 34A présente une largeur L34A et les éléments en saillie 34B une largeur L34B. La largeur L34B des éléments en saillie 34B est mesurée selon la direction latérale Y entre deux lignes parallèles à la direction longitudinale X et aux bords de la base 34A et qui comprennent tous les éléments en saillie 34B en étant tangentes aux éléments en saillie 34B. Dans le mode de réalisation de la figure 6, la largeur L34A de la base 34A est supérieure à la largeur L34B des éléments en saillie 34B, c'est-à-dire que la largeur L34B des éléments en saillie 34B est inférieure ou égale à la largeur L34A de la base 34A.

Les éléments en saillie 34B font saillie de la face supérieure 30A de l'ensemble laminé 30.

Les éléments en saillie 34B peuvent former un motif de bande anti-glissement 44 sur la bande anti-glissement 34, c'est-à-dire que la base 34A, en particulier la face supérieure 34AA de la base 34A, peut comporter des zones 46 dépourvues d'éléments en saillie 34B et une zone de la bande anti-glissement 34 munie d'éléments en saillie 34B formant le motif de bande anti-glissement 44. Le motif de bande anti-glissement 44 peut être unique ou peut être répété plusieurs fois sur la bande anti-glissement 34 selon la direction longitudinale X et/ou la direction latérale Y. Le motif de bande anti-glissement 44 peut comprendre un contour fermé.

La base 34A présente, selon la direction transversale Z, une épaisseur E34A et les éléments en saillie 34B présentent, selon la direction transversale Z, une hauteur H34B. La hauteur H34B d'un élément en saillie 34B est mesurée perpendiculairement à la face supérieure 34AA de la base, entre la base et un point de l'élément en saillie 34B le plus éloigné de la face supérieure 34AA de la base 34A.

La pluralité d'éléments en saillie 34B peut comprendre des éléments en saillie 34B présentant des hauteurs H34B différentes et/ou des largeurs L34BB différentes mesurées dans un plan parallèle au plan XY. La largeur L34BB est mesurée à l'endroit de l'élément en saillie présentant une largeur maximale.

Les éléments en saillie 34B peuvent être des picots et/ou des plots et/ou des tiges, chaque tige comportant une tête disposée à une extrémité de la tige opposée à la base 34A. Les éléments en saillie 34B peuvent être d'un seul type pour une bande anti-glissement 30 donnée ou peuvent être un mélange d'un ou plusieurs types d'éléments en saillie 34B.

Comme représenté sur la figure 7, la bande anti-glissement 34 comprend des picots 34BB présentant une hauteur H34B selon la direction transversale Z et une largeur L34BB dans un plan parallèle au plan XY. Sur la figure 7, un seul picot 34BB a été représenté. Le picot 34BB présente une hauteur H34B supérieure ou égale à la largeur L34BB. Lorsque la hauteur H34B est inférieure à la largeur L34BB, on parle alors de plot. La bande anti-glissement 34 est laminée avec une couche de support 32, formée dans ce mode de réalisation par une nappe de non-tissé dont une partie des fibres a pénétré dans la base 34A de la bande anti-glissement. On comprend donc que l'assemblage de la couche de support 32 et de la bande anti-glissement 34 a été réalisé avant solidification complète de la base 34A de la bande anti-glissement 34 de manière à faire pénétrer au moins partiellement la couche de support 32 dans la base 34A. Comme représenté sur la figure 7, il est représenté une bande anti-glissement 34 et une couche de support 32 comprenant un non-tissé. Ce non-tissé peut former une oreille avant 16 et/ou une oreille arrière 20 pour couche culotte 10, en particulier la bande anti-glissement 34 peut être agencée sur l'oreille avant 16 de telle sorte que les éléments en saillie s'étendent vers la partie absorbante de l'article absorbant et/ou s'étendent à l'opposé de la partie absorbante de l'article absorbant.

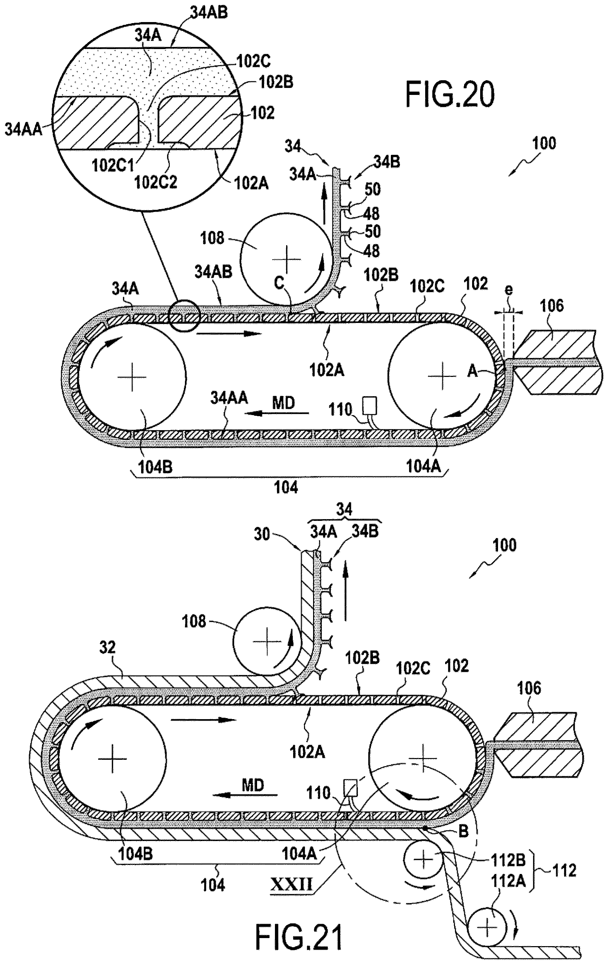

Des éléments en saillie comprenant une tige surmontée d'une tête sont représentés sur les figures 20 à 23.

La figure 2B est une vue éclatée de deux couches de support 32 selon la figure 2A et présente un mode d'assemblage possible de la couche de support 32. Sur la figure 2B, deux couches de support 32 sont présentées assemblées l'une à l'autre. Pour former la couche de support 32, une fois les nappes de non-tissé, le film élastique et les films de colle assemblés par laminage, l'ensemble de la figure 2B est coupé au milieu pour former deux couches de support 32.

Dans le mode de réalisation de la figure 2B, la couche de support 32 comprend une première nappe de non-tissé 36 et une deuxième nappe de non-tissé 38. Les première et deuxième nappes de non-tissé 36, 38 peuvent être de même nature ou être différentes.

Dans le mode de réalisation de la figure 2B, les première et deuxième nappes de non-tissé 36, 38, comprennent chacune des zones non activées 36A, 38A et des zones activées 36B, 38B préalablement à l'assemblage.

Dans le mode de réalisation de la figure 2B, la dimension selon la direction latérale Y des zones activées 36B, 38B sont égales. Elles pourraient être différentes d'une nappe à l'autre et/ou dans la même nappe. Les première et deuxième nappes de non-tissé 36, 38 pourraient également ne pas comprendre de zones activées, l'activation de la couche de support étant réalisée après assemblage avec le film élastique 40.

La colle 42 est appliquée sur les première et deuxième nappes de non-tissé 36, 38. La colle 42 est disposée en bandes pleines 42A et en filets 42B. La colle 42 forme ainsi une pluralité de lignes de colle 42B, par exemple continues dans la direction longitudinale X. Le film élastique 40 présente une largeur L40. La largeur L34A de la base 34A de la bande anti-glissement 34 est inférieure à la largeur L40 du film élastique 40. Dans l'exemple de la figure 2A, la largeur L34A de la base 34A de la bande anti-glissement 34 correspond à 25% de la largueur L40 du film élastique 40.

La bande anti-glissement 34 peut être assemblée par collage et/ou par soudure ultrason sur la face supérieure 30A de la couche de support 32. La bande anti-glissement 34 peut également être assemblée par laminage de la bande anti-glissement 34 avant solidification complète de la base 34A de la bande anti-glissement 34 de manière à faire pénétrer au moins partiellement la couche de support 32 dans la base 34A, dans le mode de réalisation de la figure 2A, la deuxième nappe de non-tissé 38.

La bande anti-glissement 34 peut être fabriquée par exemple au moyen d'un appareillage 100 tel que représenté sur la figure 20. L'appareillage 100 permet de fabriquer une bande anti-glissement 34 pour un ensemble laminé 30. La bande anti-glissement 34 comprend la base 34A continue et la pluralité d'éléments en saillie 34B. Dans le mode de réalisation de la figure 20, chaque élément en saillie 34B comprend une tige 48 surmontée d'une tête 50. La tête 50 est disposée à une extrémité de l'élément en saillie 34B opposée à la base 34A, en particulier à la face supérieure 34AA de la base 34A.

L'appareillage 100 tel que représenté comprend une bande de moulage 102 positionnée sur des moyens d'entrainement en rotation 104 comprenant ici deux rouleaux 104A, 104B, un moyen de distribution de matière 106, par exemple un injecteur, adapté pour réaliser une injection de matière de moulage élastomère.

L'ensemble formé par la bande de moulage 102 et les moyens d'entrainement en rotation 104 forme ainsi un dispositif de moulage.

L'exemple illustré comprenant deux rouleaux 104A, 104B n'est pas limitatif, le nombre et l'agencement du ou des rouleaux peuvent varier notamment afin de s'adapter à la longueur de la bande de moulage 102 et aux différents postes de l'appareillage. On pourrait par exemple utiliser trois rouleaux ou encore un seul de telle sorte que la bande de moulage est agencée sur la périphérie du seul rouleau, une telle bande de moulage formant un manchon ou « sleeve » en anglais. En particulier, un seul des deux rouleaux peut être entraîné en rotation par des moyens motorisés, par exemple le rouleau 104A, l'autre rouleau 104B étant libre, c'est à dire sans moyens motorisés, et entraîné en rotation via la bande de moulage, elle-même entraînée par le rouleau 104A. La direction de défilement de la bande de moulage définit la direction MD de la bande anti-glissement.

La bande de moulage 102 telle que présentée comprend une face interne 102A et une face externe 102B, la face interne 102A étant au contact des moyens d'entrainement en rotation 104.

Le moyen de distribution de matière 106 est disposé de manière à injecter de la matière de moulage sur la face externe 102B de la bande de moulage 102.

Plus précisément, le moyen de distribution de matière 106 est disposé en regard de la bande de moulage 102, espacé de la bande de moulage 102 de manière à définir un entrefer e indiqué sur la figure 20. On repère par la référence A la limite de la matière injectée sur la face externe 102B de la bande de moulage 102, correspondant au front arrière de la matière injectée sur la bande de moulage 102 par rapport au sens de déplacement de la bande de moulage 102.

La bande de moulage 102 est munie d'une pluralité de cavités 102C permettant la réalisation des éléments en saillie 34B de la bande anti-glissement 34.

Les cavités 102C sont chacune formées de manière à définir une tige 102C1 s'étendant depuis la face externe 102B vers la face interne 102A de la bande de moulage 102 et une tête 102C2 s'étendant entre la tige 102C1 et la face interne 102A de la bande de moulage 102.

Dans l'exemple illustré, les têtes 50 des cavités 102C débouchent sur la face interne 102A de la bande de moulage 102. Les cavités 102C sont donc traversantes. Un tel mode de réalisation n'est pas limitatif, les cavités 102C peuvent également être borgnes, et donc ne pas déboucher de la face interne 102A de la bande de moulage 102 et/ou les cavités 102C peuvent ne comporter qu'un plot ou un picot.

Les portions des cavités 102C formant les tiges 102C1 s'étendent typiquement selon une direction perpendiculaire à la face externe 102B de la bande de moulage 102. Les portions des cavités 102C formant les tiges 102C1 ont typiquement une géométrie de rotation autour d'un axe perpendiculaire à la face externe 102B de la bande de moulage 102, ou une géométrie présentant un plan de symétrie s'étendant selon une direction parallèle au sens de défilement de la bande de moulage 102 et/ou selon une direction perpendiculaire au sens de défilement de la bande de moulage 102.

Les portions des cavités 102C formant les têtes 102C2 s'étendent typiquement radialement ou transversalement par rapport à un axe perpendiculaire à la face externe 102B de la bande de moulage 102, et peuvent présenter une symétrie de rotation autour de cet axe perpendiculaire à la face externe 102B de la bande de moulage 102. Les portions des cavités 102C formant les têtes 102C2 présentent typiquement une forme sensiblement tronconique ou hexaédrique.

Les portions des cavités 102C formant les têtes 102C2 peuvent être linéaires ou incurvées, par exemple pour former des portions incurvées vers la face interne 102A ou vers la face externe 102B de la bande de moulage 102 s'étendant depuis les portions des cavités 102C formant les tiges 102C1.

Les portions des cavités 102C formant les têtes 102C2 peuvent présenter une épaisseur constante ou variable.

Dans l'exemple représenté sur les figures, les portions des cavités 102C formant les têtes 102C2 s'étendent radialement autour des portions des cavités 102C formant les tiges 102C1, et présentent une forme générale de disque.

La bande de moulage 102 peut présenter sur sa face interne 102A ou sur sa face externe 102B une texturation particulière telle que des rainures, réseau de gorges ou réseau de passage formant évent ou picots, ou être sensiblement lisse, par exemple comme décrit dans la demande WO2017187103, incorporée par référence.

La bande de moulage 102 peut être formée par une superposition de plusieurs bandes, et n'est donc pas nécessairement monobloc ou monomatière.

Le moyen de distribution de matière 106 est typiquement disposé de manière à réaliser l'injection de matériau de moulage dans la bande de moulage 102 en une section de la bande de moulage 102 où cette dernière est en appui contre un rouleau d'entrainement, en l'occurrence le rouleau d'entrainement 104A dans l'exemple représenté sur la figure 20 et/ou 21. Le rouleau d'entrainement forme alors un fond pour les cavités 102C.

Dans le cas où l'injection de matériau de moulage est réalisée alors que la bande de moulage 102 n'est pas en appui contre un rouleau d'entrainement, le moyen de distribution de matière 106 peut alors comprendre une base disposée de l'autre côté de la bande de moulage 102, de sorte que la face interne 102A de la bande de moulage 102 soit en appui contre la base lorsque l'injection de matière est réalisée, la base formant alors un fond pour les cavités 102C de la bande de moulage 102.

L'utilisation d'une bande de moulage 102 associée à des moyens d'entrainement 104 par rapport à l'utilisation de moyens de formation conventionnels tels que des rouleaux dans lesquels sont directement réalisées des cavités de moulage est avantageuse pour plusieurs raisons.

L'utilisation d'une bande de moulage 102 est notamment intéressante en termes de modularité. La bande de moulage peut en effet être retirée et remplacée facilement des moyens d'entrainement, contrairement à un rouleau massif pour lequel les opérations de démontage et remontage sont particulièrement complexes à réaliser. Un tel avantage s'observe particulièrement lorsque les deux rouleaux 104A, 104B sont fixés à un bâti d'un seul et même côté, laissant l'extrémité de l'autre côté libre pour introduire/retirer la bande de moulage. Un moyen de guidage de la bande de moulage peut également être utilisé afin d'en faciliter l'introduction et/ou le retrait. Un moyen de guidage peut comprendre un élément de mise en tension de la bande de moulage.

De plus, la réalisation d'une bande de moulage est fortement simplifiée par rapport à la réalisation d'un rouleau comprenant des cavités de moulage. De tels rouleaux sont en effet typiquement réalisés par empilement de tranches successives, nécessitant donc de multiples opérations d'usinage et entraînant des contraintes importantes lors de l'assemblage et à chaque changement de référence d'éléments en saillie et présente une masse importante nécessitant le maintien de ces rouleaux par leurs deux extrémités, ce qui complexifie par conséquent leur remplacement.

Les cavités 102C dans la bande de moulage 102 peuvent être réalisées par un procédé d'attaque chimique ou par utilisation d'un laser aux endroits où l'on souhaite former des éléments en saillie 34B. On peut également envisager de réaliser la bande de moulage 102 avec des cavités 102C réparties uniformément sur toute la bande de moulage 102 et de venir ensuite boucher les cavités 102C aux endroits où l'on souhaite former des zones 20 dépourvues d'éléments en saillie 34B. Il pourrait être utilisé une bande de moulage par exemple en nickel ou en acier inoxydable ou en acier non inoxydable.

On repère sur la figure 20 par la référence C la séparation entre la bande anti-glissement 34 et la bande de moulage 102, ce point correspondant par exemple au niveau à partir duquel la base 34A de la bande anti-glissement 34 n'est plus au contact de la bande de moulage 102. On pourra prévoir que la bande de moulage 102 embarre sur le rouleau de démoulage 108, c'est-à-dire que le rouleau de démoulage 106 forme un levier dans la bande de moulage 102 pour faciliter le démoulage des éléments en saillie.

Dans l'exemple représenté, les cavités 102C de la bande de moulage 102 sont traversantes. L'appareillage peut alors comprendre un élément, tel qu'une racle 110, positionné de manière à racler la face interne 102A de la bande de moulage 102 pour retirer au besoin le matériau de moulage excédentaire. On entend par injection, l'action de mise en forme d'une matière de moulage par voie fondue, par exemple, la distribution, l'apport, le moulage, l'injection, l'extrusion.

On peut ainsi former la bande anti-glissement 34 par distribution du matériau élastomère par le moyen de distribution de matière 106 dans les cavités 102C de la bande de moulage 102 et contre la face externe 102B de la bande de moulage 102. La bande de moulage 102 de la figures 20 présente des cavités 102C, chaque cavité étant formée de manière à définir une tige 102C1 s'étendant depuis la face externe 102B vers la face interne 102A de la bande de moulage 102 et une tête 102C2 s'étendant entre la tige 102C1 et la face interne 102A de la bande de moulage 102. La bande de moulage 102 pourrait comprendre des cavités 102 ne présentant pas de partie permettant de définir une tête 102C2. Les cavités 102C de la bande de moulage 102 peuvent également ne pas être traversantes et donc ne pas déboucher sur la face interne 102A de la bande de moulage 102.

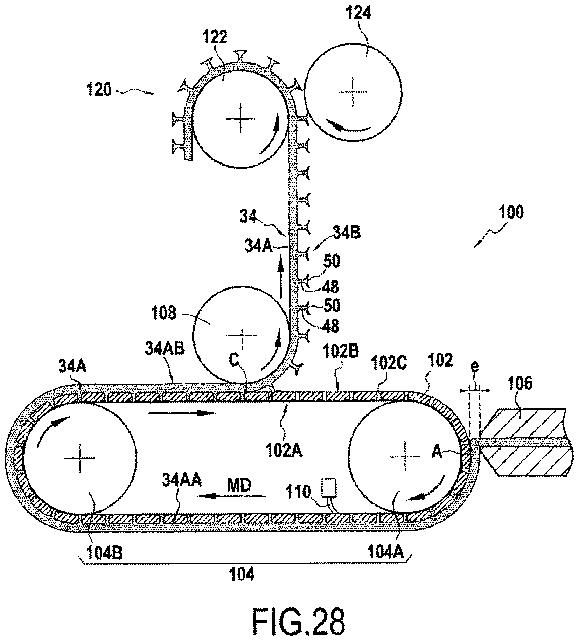

Des éléments en saillie comprenant une tige surmontée d'une tête sont représentés sur la figure 28. Ces éléments en saillie sont obtenus en calandrant les éléments en saillie présentés sur les figures 20 à 23 avec un dispositif de formage 120 comprenant un rouleau d'entrainement 122 et un rouleau de formage 124.

Lorsque l'on désire former des têtes 50 symétriques et plates, les vitesses du rouleau d'entrainement 122 et du rouleau de formage 124 sont identiques.

Lorsque l'on désire former des têtes 50 asymétriques, les vitesses du rouleau d'entrainement 122 et du rouleau de formage 124 sont différentes. Plus particulièrement, on peut utiliser le ratio suivant V122/V124 = A, avec A supérieur ou égal à 0,4, de préférence supérieur ou égal à 0,65 et inférieur ou égal à 1,6, de préférence inférieur ou égal à 1,35.

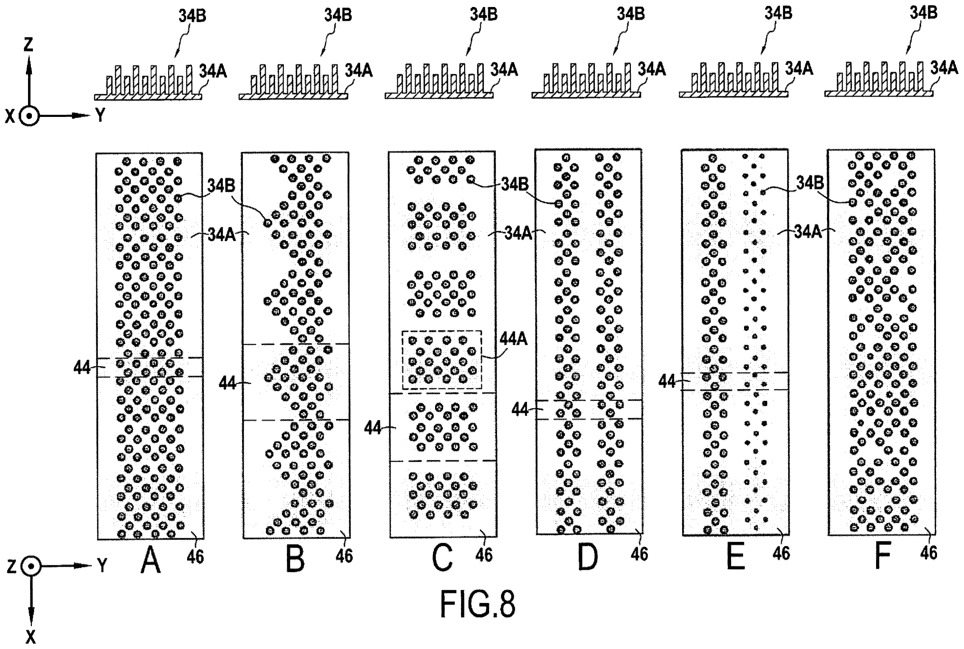

On a représenté sur les figures 8A-8F des exemples de bande anti-glissement 34. Comme on peut le constater, tous les éléments en saillie 34B ne présentent pas une hauteur H34B uniforme et peuvent présenter des largeurs L34BB différentes (voir notamment le mode de réalisation de la figure 8E). On peut également constater que les éléments en saillie 34B forment généralement un motif de bande anti-glissement 44 qui est répété selon la direction longitudinale X (voir figures 8A-8F). Le motif de la bande anti-glissement s'étend sur toute la largeur L34A de la base 34A de la bande anti-glissement 34, c'est-à-dire la dimension de la bande anti-glissement selon la direction latérale Y ou la direction CD. Cependant, la bande anti-glissement 34 peut aussi ne pas présenter de motif répétitif et être formé d'un motif de bande anti-glissement unique, comme cela est représenté sur la figure 8F. Les éléments en saillie 34B peuvent former un motif de bande anti-glissement 44 présentant un contour fermé 44A. Les éléments en saillie 34B peuvent présenter une densité différente dans la direction longitudinale X et la direction latérale Y et/ou le long de la direction longitudinale X et/ou la direction latérale Y.

La couche de support 32 peut ensuite être assemblée avec la bande anti-glissement 34 au moyen d'une colle, de soudure ultrason, et/ou via une fusion de la base 34A ou de la couche de support 32.

L'appareillage présenté précédemment et le procédé associé peuvent également présenter des moyens et une étape d'assemblage d'une couche de support 32 à la base 34A.

Afin de réaliser l'assemblage de la couche de support 32 à la base 34A de la bande anti-glissement 34, l'appareillage 100 proposé peut comprendre des moyens d'entrainement de la couche de support 32, adaptés pour réaliser une alimentation en bande et pour appliquer la couche de support 32 contre la face inférieure 34AB de la base 12 en aval du moyen de distribution de matière 106.

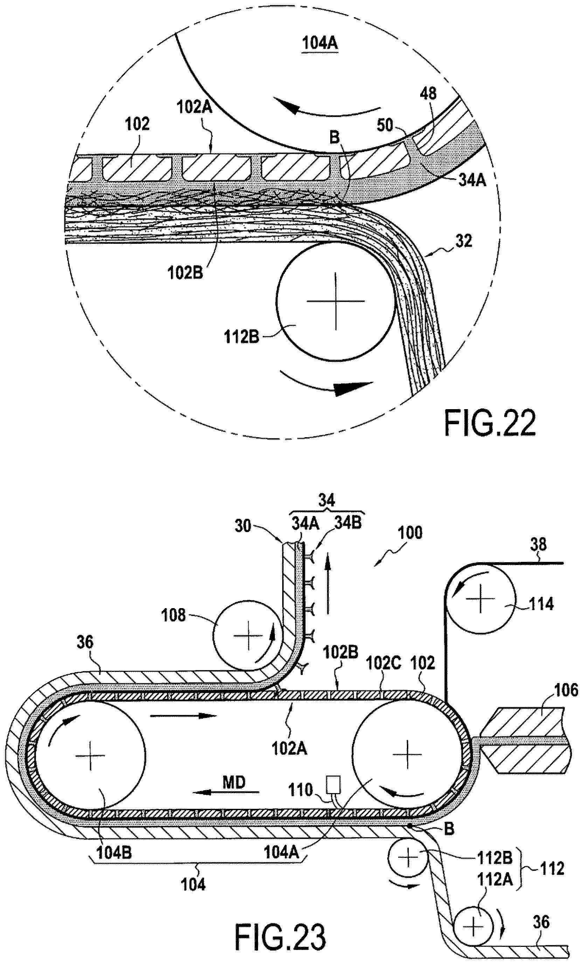

On représente schématiquement sur les figures 21 et 22 un exemple d'appareillage 100 comprenant de tels moyens. La figure 22 est une vue en détail de la zone XXII de la figure 21.

L'appareillage tel qu'illustré est similaire à celui présenté précédemment en référence à la figure 20 ; les éléments en commun ne sont donc pas décrits à nouveau ici.

Comme on le voit sur les figures 20 et 21, l'appareillage tel que présenté comprend des moyens d'entrainement de bande 112, ici constitués de deux rouleaux 112A, 112B, configurés pour réaliser une alimentation de la couche de support 32 en aval du moyen de distribution de matière 106. Dans ce mode de réalisation, la direction MD de la bande anti-glissement 34 est confondue avec la direction MD de la couche de support 32.

La couche de support 32 est typiquement une couche de matériau non-tissé, un film thermoplastique, un film élastique ou un film composite, ou encore un ensemble de fibres et/ou filaments consolidés thermiquement.

Dans l'exemple représenté sur les figures 20 et 21, la couche de support 32 est représentée comme étant une nappe de non-tissé.

Les moyens d'entraînement 112 de la couche de support 32 sont configurés pour alimenter l'appareillage en couche de support 32, et appliquer cette couche de support 32 contre la face inférieure 34AB de la base 34 en aval du moyen de distribution de matière 106.

Les moyens d'entrainement 112 sont configurés de manière à ce que cette application soit réalisée préalablement à la solidification complète de la base 34A. Ainsi, cette application entraine une pénétration au moins partielle de la couche de support 32 au-delà d'un plan défini par la face inférieure 34AB de la base 34. On repère par la référence B sur les figures le point de mise en contact entre la base 34A et la couche de support 32.

Plus précisément, la face inférieure 34AB de la base 34 est sensiblement plane, et définit un plan. L'application de la couche de support 32 contre cette face inférieure 34AB entraine une pénétration de portions de la couche de support 32, par exemple de fibres et/ou filaments de la nappe de non-tissé, dans le cas où la couche de support 32 est une nappe de non-tissé, au sein de la base 34A, traversant de ce fait la face inférieure 34AB de la base 34A.

Dans la mesure où une telle application est réalisée préalablement à la solidification complète de la base 34A, il n'est pas nécessaire de réchauffer la base 34A et/ou la couche de support 32 afin de réaliser une telle liaison.

A titre d'exemple, en considérant une base 34A réalisée en VISTAMAXX 7050 FLX (disponible chez ExxonMobil Chemical, Houston, Tex.), l'application du substrat contre la face inférieure 34AB de la base 34A est typiquement réalisée lorsque la face inférieure 34AB de la base 34A présente une température comprise entre la température de fusion du matériau et la température de ramollissement Vicat B du matériau la constituant moins 30°C ou encore entre la température de fusion du matériau la constituant et la température de ramollissement Vicat A du matériau la constituant. Plus particulièrement, lorsque la base comprend un matériau à base de polyoléfine, la face inférieure 34AB de la base 34A présente une température comprise entre 150°C et 200°C, typiquement de l'ordre de 175°C, cette température étant typiquement mesurée au moyen d'une caméra infrarouge ou laser. On entend par température de ramollissement VICAT la température obtenue selon l'une des méthodes décrites dans les normes ISO 306 ou ASTM D1525 avec une vitesse de chauffe de 50°C/h et une charge normalisée de 50N pour le VICAT B et une charge normalisée de 10N pour le VICAT A.

La couche de support 32 peut être appliquée de manière uniforme ou non uniforme contre la face inférieure 34AB de la base 34A.

La liaison réalisée entre la couche de support 32 et la base 34A peut être réalisée de manière uniforme ou non uniforme.

Dans le cas où la couche de support 32 est un ensemble de fibres et/ou filaments consolidés thermiquement, la liaison avec la base 34A est également réalisée par pénétration dans la base 34A d'une partie des fibres et/ou filaments de la couche de support 32.

Dans le cas où la couche de support 32 est une nappe de non-tissé, le démoulage des éléments en saillie est réalisé de manière aisée même avec un non-tissé dont le grammage est inférieur à 80 g/m² (masse de matière en gramme par mètre carré de non-tissé). A titre d'exemple, le grammage du non-tissé peut être compris entre 5 g/m² et 120 g/m², ou encore entre 25 g/m² et 100 g/m², ou encore entre 10 g/m² et 70 g/m².

Ce mode de solidarisation d'une couche de support 32 à une base 34A comprenant des éléments en saillie 34B est notamment avantageux en ce qu'il n'entraine pas une déformation de la base 34A, et permet donc avantageusement de conserver la forme de la base 34A obtenue lors de l'étape d'injection, et notamment de conserver les bords droits pouvant être obtenus via le procédé et l'appareillage décrits précédemment.

Dans le cas où la couche de support 32 est une nappe de non-tissé, l'appareillage peut comprendre un dispositif de calandrage en amont des moyens d'entrainement 112, permettant ainsi de réaliser une étape de calandrage localement ou non de la nappe de matériau non-tissé préalablement à son application contre la base 34A.

L'appareil 100 des figures 21 et 22 peut être utilisé pour assembler par laminage la bande anti-glissement 34 et la couche de support 32, la couche de support 32 pouvant comprendre une ou plusieurs nappes de non-tissé avec ou sans film thermoplastique (élastique ou non-élastique), comme par exemple la couche de support 32 de la figure 2A.

L'appareil 100 des figures 21 et 22 peut également être utilisé pour assembler la bande anti-glissement 34 à la deuxième nappe de non-tissé 38 de la figure 2A, la deuxième nappe de non-tissé 38 et la bande anti-glissement 34 étant ensuite assemblées au film élastique 40 et à la première nappe de non-tissé 36 de la figure 2A.

Le mode de réalisation de la figure 3 diffère du mode de réalisation de la figure 2A notamment en ce que la bande anti-glissement 34 traverse la deuxième nappe de non-tissé 38, les éléments en saillie 34B faisant saillie de la face supérieure 30A de l'ensemble laminé 30.

Dans ce mode de réalisation, la bande anti-glissement 34 est formée par distribution du matériau élastomère dans les cavités 102C de la bande de moulage 102 à travers la deuxième nappe de non-tissé 38. L'appareillage 100 comprend, comme pour l'appareillage de la figure 23, des moyens d'entrainement 114 configurés pour réaliser une alimentation de la deuxième nappe de non-tissé 38 en amont du moyen de distribution de matière 106. La bande anti-glissement 34 étant injectée à travers la deuxième nappe de non-tissé 38, cela entraine une pénétration de portions de la deuxième nappe de non-tissé 38 au sein de la base 34A. Sur la figure 23, pour des raisons de simplicité la deuxième nappe de non-tissé 38 a été représentée avec une épaisseur plus faible que l'épaisseur représentée de la première nappe de non-tissé 36. L'épaisseur des première et deuxième nappes de non-tissés 36, 38 pourrait être similaire ou différente en fonction de l'application envisagée.

Dans la mesure où une telle application est réalisée préalablement à la solidification complète de la base 34A, il n'est pas nécessaire de réchauffer la base 34A et/ou la couche de support 32 afin de réaliser une telle liaison.

La deuxième nappe de non-tissé 38 et la bande anti-glissement 34 sont ensuite assemblées au film élastique 40 et à la première nappe de non-tissé 36 de la figure 3. Le matériau de la base et le matériau du film élastique peuvent être identiques ou différents tout en restant des matériaux élastomères.

Dans le mode de réalisation de la figure 4, le film élastique 40 est en matériau élastomère. Le matériau élastomère du film élastique est par exemple le même que le matériau élastomère de la bande anti-glissement et les première et deuxième nappes de non-tissé 36, 38 sont assemblées par laminage au film élastique 40 sans addition de colle.

Typiquement, les première et deuxième nappes 36, 38 peuvent être assemblées au film élastique 40 avant solidification complète du film élastique 40 par application de la première nappe de non-tissé 38 contre la face inférieure 34AB de la base 34A et de la deuxième nappe de non-tissé 36 contre la face supérieure 34AA de la base 34A, ce qui entraine une pénétration de portions des première et deuxième nappes de non-tissé 36, 38 au sein de la base 34A.

Dans la mesure où une telle application est réalisée préalablement à la solidification complète de la base 34A, il n'est pas nécessaire de réchauffer la base 34A et/ou la couche de support 32 afin de réaliser une telle liaison. Le matériau de la base et le matériau du film élastique peuvent être identiques ou différents tout en restant des matériaux élastomères. Selon une variante de réalisation non représentée, la première nappe de non-tissé pourrait être liée au film élastique via une couche de colle (en continu et/ou sous forme de lignes de colle, par exemple comme représenté à la figue 3).

L'appareillage 100 de la figure 23 comprend des moyens d'entrainement 112, 114 configurés respectivement pour réaliser une alimentation de la première nappe de non-tissé 36 en aval du moyen de distribution de matière 106 et pour réaliser une alimentation de la deuxième nappe de non-tissé 38 en amont du moyen de distribution de matière 106.

Lorsque la matière élastomère de la bande anti-glissement 34 est différente de la matière élastomère du film élastique 40 ou lorsque le film 40 est un film thermoplastique non élastique, l'appareillage de la figure 23 comprend un deuxième moyen de distribution de matière disposé en aval du moyen de distribution de matière 106.

Dans le mode de réalisation de la figure 5, la deuxième nappe de non-tissé 38 est assemblée par collage au moyen d'une colle 42 sur le film élastique 40. La deuxième nappe de non-tissé 38 est en deux parties, chaque partie étant disposée de part et d'autre de la bande anti-glissement 34. Le matériau de la base et le matériau du film élastique peuvent être identiques ou différents tout en restant des matériaux élastomères. Selon une variante de réalisation non représentée, la première nappe de non-tissé pourrait être liée au film élastique via une couche de colle (en continue et/ou sous forme de lignes de colle, par exemple comme représenté à la figue 3).

Dans les modes de réalisation des figures 2 à 4, la bande anti-glissement est disposée au milieu selon la direction latérale Y de la couche de support 32. Dans le mode de réalisation de la figure 5, la bande anti-glissement n'est pas centrée.

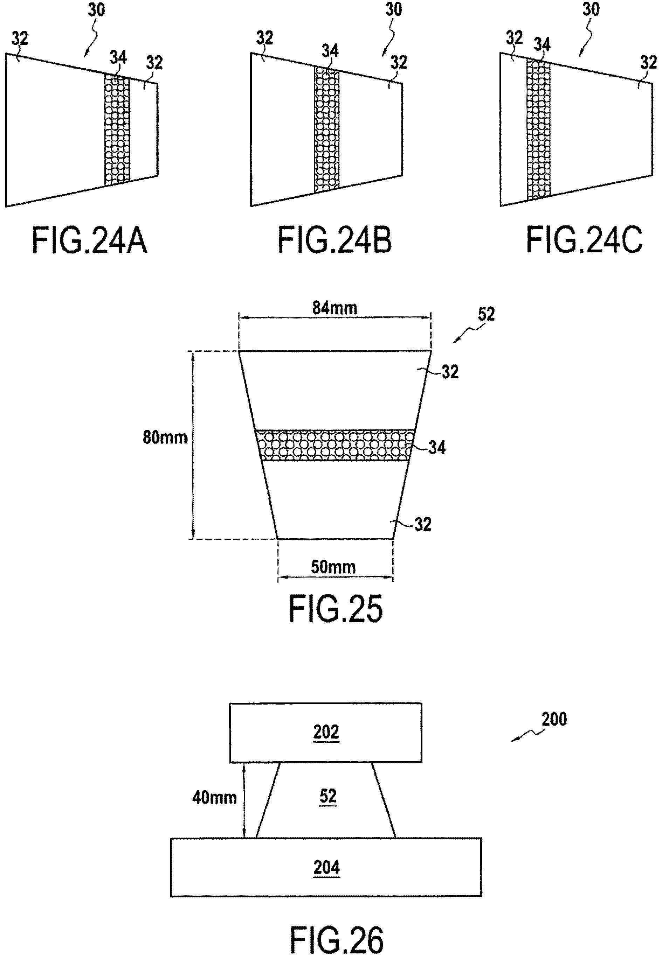

La bande anti-glissement 34 peut être disposée à différents endroits de la couche de support 32, comme représenté sur les figures 24A-24B. La bande anti-glissement peut être disposée dans toute position intermédiaire entre les positions des figures 24A-24C. Sur les figures 24A-24C, la bande anti-glissement 34 présente une dimension selon la direction MD qui est plus importante qu'une dimension selon la direction CD, on comprend que la bande anti-glissement présente une longueur selon la direction MD et une largeur selon la direction CD.

Les bandes de moulage 102 des figures 20 à 23 présentent des cavités 102C, chaque cavité étant formée de manière à définir une tige 102C1 s'étendant depuis la face externe 102B vers la face interne 102A de la bande de moulage 102 et une tête 102C2 s'étendant entre la tige 102C1 et la face interne 102A de la bande de moulage 102. La bande de moulage 102 pourrait comprendre des cavités 102 ne présentant pas de partie permettant de définir une tête 102C2. Les cavités 102C de la bande de moulage 102 peuvent également ne pas être traversantes et donc ne pas déboucher sur la face interne 102A de la bande de moulage 102.

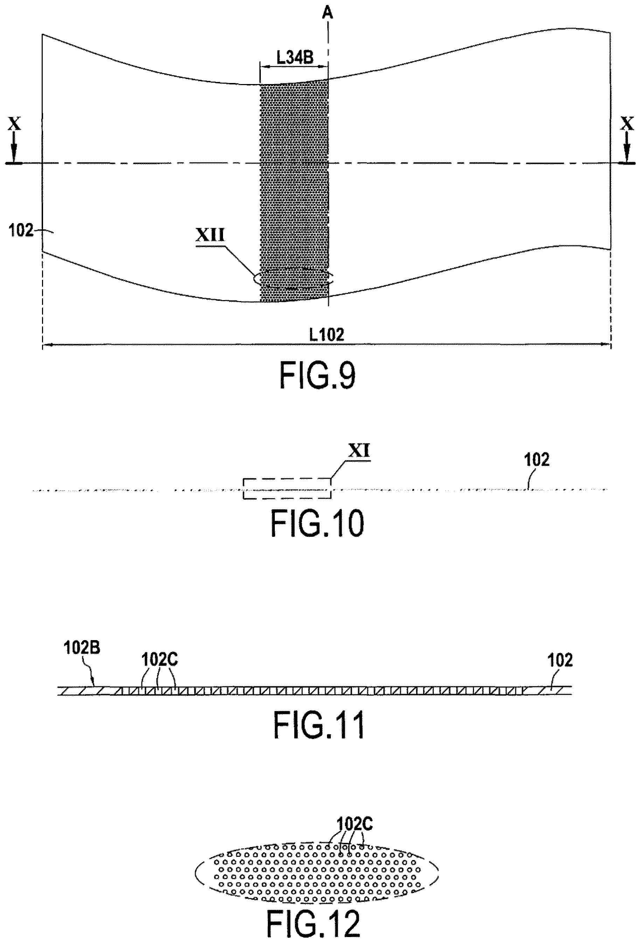

Les figures 9 à 19 représentent des bandes de moulage 102 permettant de former des bandes anti-glissement 34 présentant des motifs variés.

La figure 9 est une vue partielle d'une bande de moulage 102 dont la largeur L102 selon la direction latérale Y est supérieure à la largeur L34B du motif des éléments en saillie 34B. La bande de moulage 102 est vue par la face externe 102B. La figure 10 est une vue en coupe selon le plan de coupe X-X de la figure 9 et la figure 11 est un agrandissement XI de la figure 10 montrant les cavités 102C de moulage de la bande de moulage 102. La figure 12 est une vue agrandie de la figure 9 montrant les cavités 102C de la bande de moulage 102. On notera que dans le mode de réalisation de la figure 9, la partie perforée de la bande de moulage 102 n'est pas centrée par rapport à l'axe de symétrie A de la bande de moulage 102. La partie perforée de la bande de moulage 102, correspondant à la largeur L34B des éléments en saillie 34B sur la base 34A de la bande anti-glissement 34 pourrait être centrée sur l'axe de symétrie A, voire décalée vers la gauche ou la droite de la figure 9.

Par exemple, la bande anti-glissement 34 obtenue par distribution de matière élastomère avec la bande de moulage 102 des figures 9 à 12 est une bande anti-glissement dont la largeur L34A mesurée selon la direction latérale Y est égale à 20 mm. En prenant toute la largeur L34A de la bande anti-glissement, une surface de 22,52 mm² comprend 49 éléments en saillie 34B, soit une densité de 217,616 éléments en saillie par cm², représentant 13,40% de la surface totale de la base 34A.

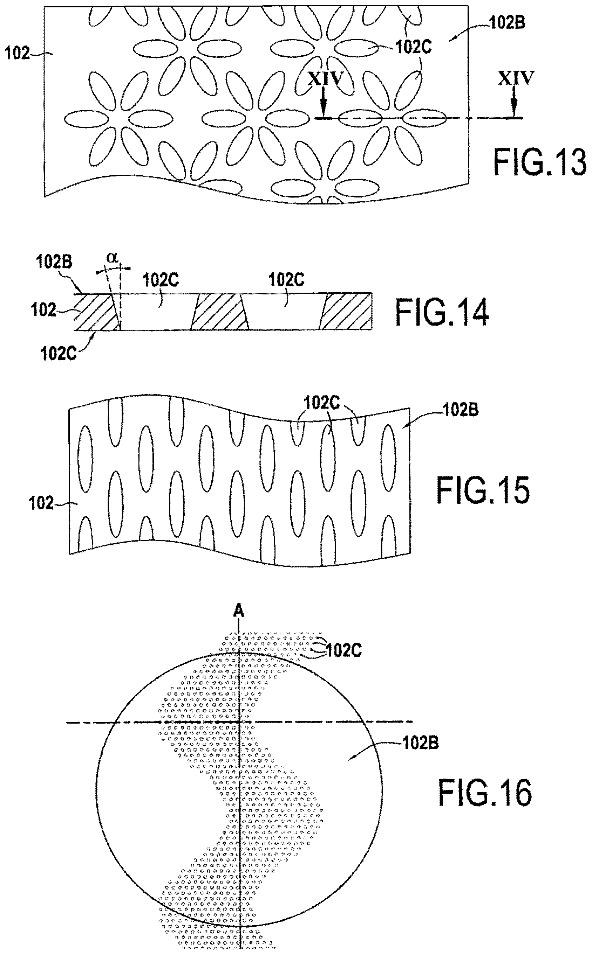

La figure 13 est une vue d'une bande de moulage 102 similaire à la vue de la figure 12 et la figure 14 est une vue en coupe selon le plan XIV similaire à la vue de la figure 11 pour le motif de la figure 13. La figure 13 présente un motif en « fleur » des cavités 102C de la bande de moulage 102. Comme représenté sur la figure 14, les cavités 102 peuvent présenter une dépouille, ici un angle α inférieur ou égale à 10° qui permet de faciliter le démoulage des éléments en saillie.

Par exemple, la bande anti-glissement 34 obtenue par distribution de matière élastomère avec la bande de moulage 102 des figures 13 et 14 est une bande anti-glissement dont la largeur L34A mesurée selon la direction latérale Y est égale à 20 mm. En prenant toute la largeur L34A de la bande anti-glissement, une surface de 130 mm² comprend 30 éléments en saillie 34B, soit une densité de 23,077 éléments en saillie par cm², représentant 29,05% de la surface totale de la base 34A.

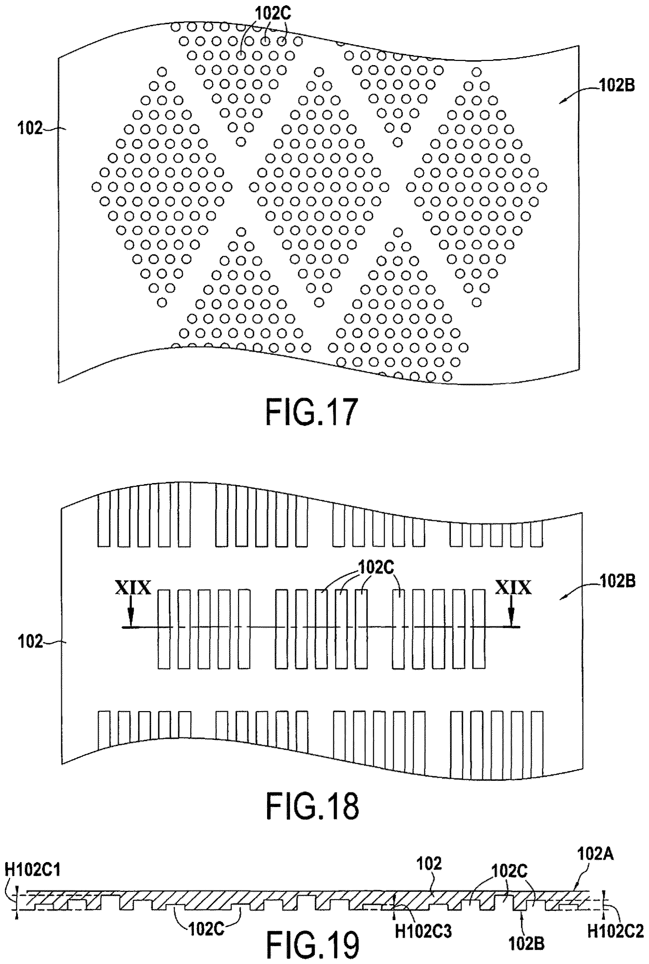

Les figures 15 à 19, qui sont des vues similaires à la vue de la figure 12 représentent d'autres motifs formés par les cavités 102C de bandes de moulage 102. La figure 19 est une vue similaire à la vue de la figure 14 pour le motif de la figure 18. Comme on peut le voir, les cavités 102C ne sont pas traversantes et ne présentent pas une profondeur uniforme. Les cavités 102C présentent trois hauteurs H102C1, H102C2, H102C3 différentes.

Par exemple, la bande anti-glissement 34 obtenue par distribution de matière élastomère avec la bande de moulage 102 de la figure 15 est une bande anti-glissement dont la largeur L34A mesurée selon la direction latérale Y est égale à 20 mm. En prenant toute la largeur L34A de la bande anti-glissement, une surface de 110 mm² comprend 11 éléments en saillie 34B, soit une densité de 10 éléments en saillie par cm², représentant 25,15% de la surface totale de la base 34A.

Par exemple, la bande anti-glissement 34 obtenue par distribution de matière élastomère avec la bande de moulage 102 de la figure 16 est une bande anti-glissement dont la largeur L34A mesurée selon la direction latérale Y est égale à 20 mm. En prenant toute la largeur L34A de la bande anti-glissement, une surface de 382,78 mm² comprend 225 éléments en saillie 34B, soit une densité de 58,78 éléments en saillie par cm², représentant 8,11% de la surface totale de la base 34A.

Par exemple, la bande anti-glissement 34 obtenue par distribution de matière élastomère avec la bande de moulage 102 de la figure 17 est une bande anti-glissement dont la largeur L34A mesurée selon la direction latérale Y est égale à 20 mm. En prenant toute la largeur L34A de la bande anti-glissement, une surface de 251,66 mm² comprend 405 éléments en saillie 34B, soit une densité de 160,93 éléments en saillie par cm², représentant 9,91% de la surface totale de la base 34A.

Par exemple, la bande anti-glissement 34 obtenue par distribution de matière élastomère avec la bande de moulage 102 de la figure 18 est une bande anti-glissement dont la largeur L34A mesurée selon la direction latérale Y est égale à 20 mm. En prenant toute la largeur L34A de la bande anti-glissement, une surface de 187,35 mm² comprend 35 éléments en saillie 34B, soit une densité de 18,682 éléments en saillie par cm², représentant 25,22% de la surface totale de la base 34A.

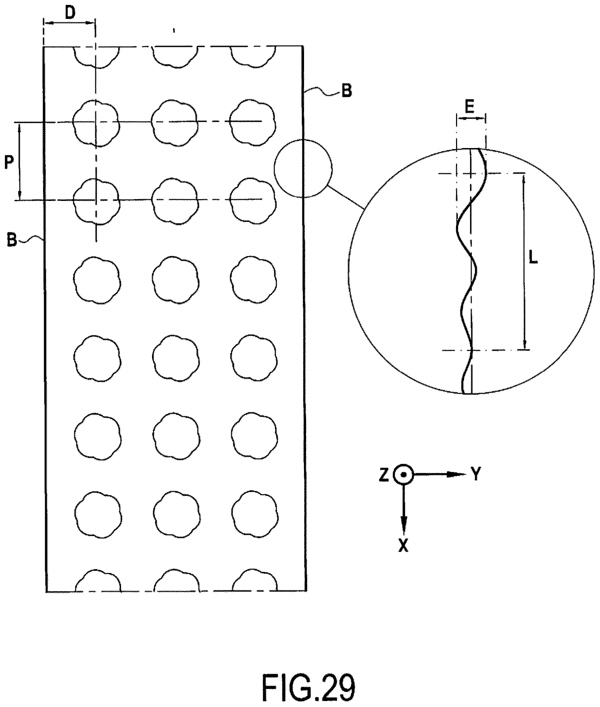

Comme représenté sur la figure 29, la bande anti-glissement 34 est obtenue suite à l'injection de matière élastomère dans la bande de moulage 102, la bande anti-glissement 34 s'étend donc selon la direction longitudinale X sur la figure 29. On représente également sur la figure 29 la direction latérale Y. La direction longitudinale X est ici parallèle à la direction machine MD, c'est-à-dire la direction d'entrainement de la bande anti-glissement 34.

On définit pour cette bande anti-glissement 34 deux bords B s'étendant chacun selon la direction longitudinale X, ces deux bords B définissant les deux extrémités de la base 34A de la bande anti-glissement 34 selon la direction latérale Y, orthogonale à la direction longitudinale X.

Les éléments en saillie sont généralement agencés à proximité des bords B, par exemple à une distance D des bords B comprise entre 2 et 3 pas P d'éléments en saillie, typiquement égale à 2 ou 3 pas P, la distance D étant mesurée selon la direction latérale Y par rapport à la direction longitudinale X. Le pas P entre deux éléments en saillie correspond à la distance entre deux éléments en saillie successifs selon la direction longitudinale. Dans l'exemple représenté sur la figure 29, les éléments en saillie sont agencés en colonnes s'étendant selon la direction longitudinale X, ces colonnes étant répétées à l'identique selon la direction latérale Y. Les éléments en saillie peuvent également être agencés en quinconce ou « nid d'abeille », par exemple en décalant les colonnes des éléments en saillie selon la direction longitudinale.

Comme représentée sur la figure 29, chacun des bords B présente une succession de monts et de vallées, ladite succession s'étendant selon la direction longitudinale L et lesdits monts et vallées s'étendant dans un plan parallèle à celui formé par la base 34A de la bande anti-glissement 34, ces monts et vallées traduisant de légères irrégularités dans la distribution de matière de moulage pour la formation de la bande anti-glissement 34, étant entendu qu'un bord parfaitement rectiligne n'est pas réalisable industriellement.

Les vallées sont entendues comme étant les régions des bords B faisant saillie vers l'intérieur de la bande anti-glissement 34tandis que les monts sont entendus comme étant les régions des bords B faisant saillie vers l'extérieur de la bande anti-glissement 34.

La régularité des bords B peut donc s'évaluer grâce à ces monts et vallées successifs.

Les bords B présentent, en vue en coupe selon une direction transversale à la direction longitudinale, une portion de forme arrondie. Plus particulièrement, la forme arrondie est orientée à l'extérieur latéral de la base 34A. Cette forme arrondie est réalisée lors de la formation de la base 34A. En d'autres termes, cette forme arrondie n'a pas été obtenue par une découpe.

L'appareillage et le procédé tels que présentés précédemment permettent d'obtenir des bords B de la bande anti-glissement 34 tels que pour une longueur L selon la direction longitudinale L correspondant à trois monts consécutifs, l'écart E maximum entre les monts et les vallées selon la direction latérale Y, orthogonale à la direction longitudinale X est inférieur à 3 mm, ou plus précisément inférieur à 2 mm, ou encore plus précisément inférieur à 1 mm, ou encore compris entre 0,001 mm et 1 mm, plus particulièrement entre 0,001 mm et 0,5 mm, encore plus particulièrement entre 0,001 mm et 0,1 mm.

Une telle définition est également applicable pour une longueur correspondant à trois vallées consécutives ; l'écart maximum entre les monts et les vallées selon la direction latérale Y est inférieur à 3 mm, ou plus précisément inférieur à 2 mm, ou encore plus précisément inférieur à 1 mm, ou encore compris entre 0,001 mm et 1 mm, plus particulièrement entre 0,001 mm et 0,5 mm, encore plus particulièrement entre 0,001 mm et 0,1 mm.

Les 3 monts ou vallées consécutifs sont typiquement sur une distance inférieure à la distance correspondant à 15 pas P d'éléments en saillie, de préférence inférieure à une distance de 25 mm.

L'obtention de bords B que l'on peut ainsi qualifier de « droits » est avantageuse en ce qu'elle permet de s'affranchir d'une étape ultérieure de rectification des bords, par exemple via une étape de découpe, de tels bords droits étant perçus par l'utilisateur comme un signe de qualité du produit.

Par ailleurs, l'appareillage et le procédé employés permettent d'obtenir de tels bords droits sans nécessiter la formation de surépaisseurs en bordure de ruban, de telles surépaisseurs ne présentant pas d'intérêt fonctionnel.

Comme on le comprend de la description qui précède, les bords droits sont obtenus via l'injection du matériau de moulage par le moyen de distribution de matière 106. Les étapes ultérieures de démoulage et de formage conservent ces bords droits tels que décrits précédemment, dans la mesure où ces étapes n'entrainent pas l'application d'efforts sur les bords de la base 34A de la bande anti-glissement 34. La bande anti-glissement 34 ainsi obtenue à l'issue de ces différentes étapes présente donc un bord droit tel que défini précédemment.

Pour les mesures de coefficient de frottement statique, de déformation résiduelle et d'allongement à la rupture, les échantillons de mesure sont préparés de manière similaire et selon la méthode décrite ci-après.

L'ensemble laminé est conditionné dans une atmosphère normale, telle que définie dans la norme ASTM D5170, à une température de 23°C +/- 2°C et un taux d'humidité relative de 50% +/- 5%pendant 24 heures.

Le coefficient de frottement statique est mesuré, conformément à la norme ASTM D1894, en déplaçant un patin de 200 g (gramme) présentant une surface de 63 mm x 63 mm, à une vitesse de 150 mm/min (millimètre par minute) sur la surface de la bande anti-glissement.

Dans une première partie de l'essai, un échantillon de l'ensemble laminé a été fixé sur la table de frottement du système d'essai dans une position absolument plate à l'aide du ruban adhésif. Les types de ruban qui peuvent être utilisés pour coller les échantillons des matériaux à la table de friction sont bien connus des personnes ayant des compétences ordinaires dans l'art et ne seront donc pas décrits plus en détail dans le présent document.

Dans une autre partie de l'essai, l'oreille avant formée d'un non-tissé Spunbond (60 gsm PP (gram per square centimeter - gramme par centimètre carré) de la marque ULTRATEX D 1A 60 X disponible auprès de la société « TEXBOND nonwovens » est positionnée sur la face inférieure du patin en regard de la face supérieure de l'échantillon de l'ensemble laminé, la face calandrée du Spunbond étant en regard de la face supérieure de l'échantillon de l'ensemble laminé. Le patin est animé en translation sur l'échantillon à tester sur au moins une longueur de 15 mm permettant d'obtenir la valeur de coefficient de frottement statique.

Pour la mesure du coefficient de frottement statique à 15% d'élongation, l'échantillon de l'ensemble laminé 30 est maintenu à 15% d'élongation de la bande anti-glissement 34 par des adhésifs double face. La méthode de mesure est la même que pour la mesure du coefficient de frottement statique au repos.

Pour un échantillon d'ensemble laminé dépourvu de bande anti-glissement, par exemple un ensemble laminé commercialisé sous le nom « High Stretch Surefit 100 » sous la référence FM27R0140N010-AS02N, disponible auprès de la société « APLIX », le coefficient de frottement statique est de 0,37 dans la direction MD et 0,42 dans la direction CD au repos.

Pour un ensemble laminé comprenant une couche de support « High Stretch Surefit 100 » et une bande anti-glissement obtenue au moyen de la bande de moulage 102 de la figure 9, le coefficient de frottement statique est de 0,53 dans la direction MD et 0,88 dans la direction CD au repos et de 0,73 dans la direction MD et 0,99 dans la direction CD lorsque l'échantillon est étiré de 15% dans la direction CD.

Pour un ensemble laminé comprenant une couche de support « High Stretch Surefit 100 » et une bande anti-glissement obtenue au moyen de la bande de moulage 102 de la figure 13, le coefficient de frottement statique est de 0,71 dans la direction MD et 1,06 dans la direction CD au repos et de 0,57 dans la direction MD et 1,07 dans la direction CD lorsque l'échantillon est étiré de 15% dans la direction CD.

Pour un ensemble laminé comprenant une couche de support « High Stretch Surefit 100 » et une bande anti-glissement obtenue au moyen de la bande de moulage 102 de la figure 15, le coefficient de frottement statique est de 0,54 dans la direction MD et 0,72 dans la direction CD au repos et de 0,79 dans la direction MD et 0,84 dans la direction CD lorsque l'échantillon est étiré de 15% dans la direction CD.

Pour les mesures de déformation résiduelle et d'allongement à la rupture, l'équipement de mesure est un dynamomètre conforme à la norme EN 10002, par exemple le Synergie 200H, 1 colonne disponible auprès de la société MTS Systems Corp., USA, conjointement avec un logiciel d'utilisation TESTWORKS 4.04B.

Un échantillon 52 est découpé au cutter ou aux ciseaux à la forme désirée, voir figure 25. L'échantillon 52 présente une forme générale de trapèze isocèle.

La bande anti-glissement 34 présente une longueur et une largeur. Lorsque la longueur de la bande anti-glissement est parallèle à la direction MD, l'échantillon 52 présente, selon la direction CD une dimension de 80 mm. La petite et la grande base du trapèze isocèle sont parallèles à la direction MD et mesurent respectivement 50 mm et 84 mm, comme représenté sur la figure 25.

Chaque bord de l'échantillon 52 est renforcé avec un renfort fixé sur l'échantillon avec un adhésif double face. Chaque bord renforcé de l'échantillon 52 est ensuite disposé dans une mâchoire 202, 204 du dynamomètre 200 voir figure 26). Avant essai, l'écart entre les deux mâchoires 202, 204 est de 40 mm.

Les essais d'allongement à la rupture sont réalisés à vitesse de déplacement constante des mâchoires 202, 204 l'une par rapport à l'autre. Généralement, une mâchoire est fixe, ici la mâchoire inférieure 204 et l'autre mâchoire est mobile, ici la mâchoire supérieure 202. Pour réaliser l'essai d'élongation à la rupture, on déplace la mâchoire mobile à une vitesse constante de 508 mm/min jusqu'à détection de la rupture.

Les essais d'allongement à la rupture permettent d'obtenir l'allongement à 10 N (newton) exprimé en pourcentage de la dimension initiale de l'échantillon 52 ou en mm, l'allongement à la rupture exprimée en pourcentage de la dimension initiale de l'échantillon 52 ou en mm et la force à la rupture exprimée en N.

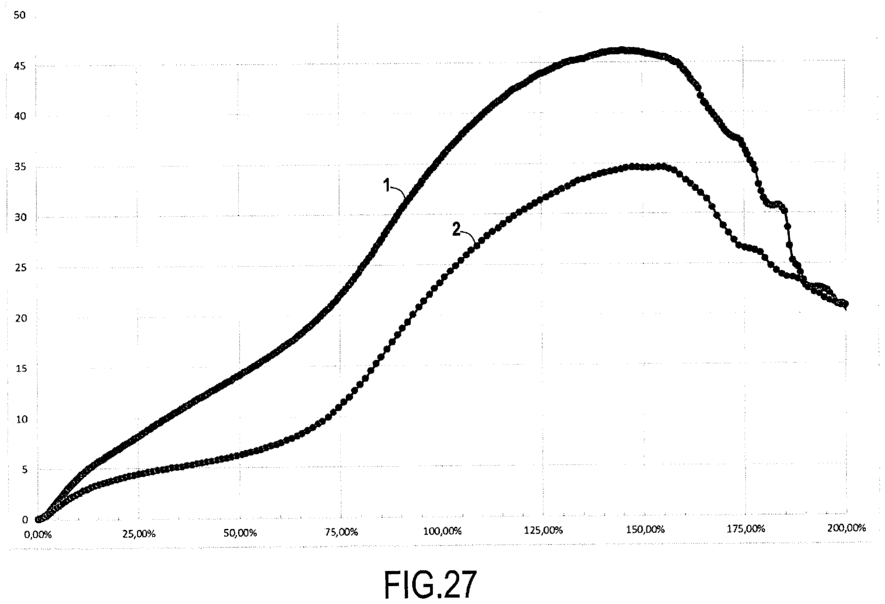

Des courbes d'essai d'allongement à la rupture sont représentées à la figure 27. Sur le graphe de la figure 27, l'abscisse représente l'allongement exprimé en % de la dimension initiale de l'échantillon 52 et l'ordonnée représente la force exprimée en N. La courbe 1 représente l'essai d'allongement à la rupture pour un ensemble laminé comprenant une bande anti-glissement et la courbe 2 représente l'essai d'allongement à la rupture de la couche de support de l'ensemble laminé de la courbe 1, c'est-à-dire un ensemble laminé dépourvu de bande anti-glissement. On constate que l'allongement à la rupture est sensiblement similaire pour les courbes 1 et 2. Par contre, la force requise pour arriver à la rupture de l'échantillon 52 est supérieure lorsque la couche de support comprend une bande anti-glissement. Pour tout allongement avant la force maximale, la force obtenue pour un ensemble laminé comprenant une bande anti-glissement est supérieure à la force obtenue pour un ensemble laminé dépourvu d'une bande anti-glissement.

Les essais de déformation résiduelle sont réalisés avec le même type d'échantillon que les échantillons 52 des essais d'allongement à la rupture et sur le même équipement.

La vitesse de déplacement la mâchoire mobile est une vitesse constante de 508 mm/min, l'écart initial des mâchoires est de 40 mm et on réalise un étirement de l'échantillon jusqu'à atteindre une force de 10 N. Une fois la force de 10 N atteinte, on arrête le déplacement de la mâchoire mobile et on maintient l'écartement pendant 30 secondes. On déplace ensuite la mâchoire mobile à vitesse constante vers sa position de départ et on maintient l'échantillon 52 pendant 60 secondes dans cette position.

On obtient ainsi une courbe donnant la force d'étirement exprimée en N en fonction de l'allongement exprimé en % de la dimension initiale de l'échantillon. Cette courbe présente une hystérésis qui permet de déterminer la déformation résiduelle ou SET à la fin d'un cycle de la manière suivante : SET = point d'intersection avec l'axe des X de la courbe mesurée pendant le déplacement de la mâchoire mobile lorsque la mâchoire mobile retourne vers sa position de départ, c'est-à-dire un écartement des mâchoires de 40 mm.

Quoique le présent exposé ait été décrit en se référant à un exemple de réalisation spécifique, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif. Par exemple, la bande anti-glissement peut présenter une direction MD qui n'est pas parallèle à la direction MD de la couche de support. En variante de réalisation des modes de réalisation des figures 2A à 8F, 20 à 25 et 28, on pourrait avoir des plots et/ou des picots et/ou des tiges surmontées de têtes selon les figures 20 à 23 et/ou des tiges surmontées de têtes selon la figure 28.

## Revendications

1. Article absorbant, tel qu'une couche culotte jetable, comprenant un ensemble laminé (30) comprenant une couche de support (32) et une bande anti-glissement (34), la bande anti-glissement (34) comprenant un matériau élastomère, la couche de support (32) et la bande anti-glissement (34) étant laminées ensemble, la bande anti-glissement (34) comprenant une base (34A) et une pluralité d'éléments en saillie (34B) s'étendant de la base (34A) et la pluralité d'élément en saillie (34B) faisant saillie d'une face (30A) de l'ensemble laminé (30).

2. Article absorbant selon la revendication 1, dans lequel la couche de support (32) comprend une nappe de non-tissé (36, 38).

3. Article absorbant selon la revendication 1 ou 2, dans lequel la couche de support (32) comprend un film thermoplastique.

4. Article absorbant selon la revendication 3, dans lequel le film thermoplastique est un film élastique (40).

5. Article absorbant selon la revendication 4, dans lequel la base (34A) de la bande anti-glissement (34) et le film élastique (40) présentent chacun une largeur, la largeur (L34A) de la base (34A) étant inférieure à la largeur (L40) du film élastique (40), de préférence la largeur de la base étant supérieure ou égale à 10% de la largeur du film élastique et inférieure ou égale à 60% de largeur du film élastique.

6. Article absorbant selon la revendication 4 ou 5, dans lequel la couche de support (32) comprend une première nappe de non-tissé (36), une deuxième nappe de non-tissé (38) et le film élastique (40), le film élastique (40) étant laminé entre les première et deuxième nappes de non-tissé (36, 38).

7. Article absorbant selon l'une quelconque des revendications 1 à 6, dans lequel, au repos, dans une zone comprenant les éléments en saillie (34B), la bande anti-glissement (34) présente un coefficient de frottement statique, mesuré selon la norme ASTM D1894, selon la direction MD et/ou la direction CD, supérieur ou égal à 0,1, de préférence supérieur ou égal à 0,5, encore plus de préférence supérieur ou égal à 0,8 et inférieur ou égal à 10, de préférence inférieur ou égal à 5, encore plus de préférence inférieur ou égal à 3.

8. Article absorbant selon l'une quelconque des revendications 1 à 7, dans lequel la bande anti-glissement (34) présente un coefficient de frottement statique, mesuré selon la norme ASTM D1894, lorsque la bande anti-glissement (34) est étirée à 15% de la valeur au repos, compris entre 50% et 150% du coefficient de frottement statique au repos.

9. Article absorbant selon l'une quelconque des revendications 1 à 8, dans lequel, au repos, la pluralité d'éléments en saillie (34B) présente une densité d'éléments en saillie supérieure ou égale à 3 éléments en saillie par cm², de préférence supérieure ou égale à 10 éléments en saillie par cm² et inférieure ou égale à 400 éléments en saillie par cm², de préférence inférieure ou égale à 300 éléments en saillie par cm².

10. Article absorbant selon l'une quelconque des revendications 1 à 9, dans lequel, au repos, la pluralité d'éléments en saillie (34B) présente un motif comprenant une répétition d'un motif de bande anti-glissement (44).

11. Article absorbant selon l'une quelconque des revendications 1 à 10, dans lequel une somme des surfaces définies sur la base (34A) par les projections orthogonales des éléments en saillie (34B) sur la base (34A), est supérieure ou égale à 1% de la surface totale de la base du motif de bande anti-glissement (44), de préférence supérieure ou égale à 5% et inférieure ou égale à 40% de la surface totale de la base du motif de bande anti-glissement (44), de préférence inférieure ou égale à 35%.

12. Article absorbant selon l'une quelconque des revendications 1 à 11, dans lequel la base (34A) présente une épaisseur (E34A) supérieure ou égale à 10 µm, de préférence supérieure ou égale à 15 µm et inférieure ou égale à 200 µm, de préférence inférieure ou égale à 150 µm.

13. Article absorbant selon l'une quelconque des revendications 1 à 12, dans lequel les éléments en saillie (34A) sont des picots et/ou des plots et/ou des tiges, chaque tige comportant une tête disposée à une extrémité de la tige opposée à la base.

## Patentansprüche

1. Absorbierender Artikel, wie eine Wegwerfwindel, umfassend eine laminierte Anordnung (30), die eine Trägerschicht (32) und einen Antirutschstreifen (34) umfasst, wobei der Antirutschstreifen (34) ein Elastomermaterial umfasst, wobei die Trägerschicht (32) und der Antirutschstreifen (34) zusammenlaminiert sind, wobei der Antirutschstreifen (34) eine Basis (34A) und mehrere vorstehende Elemente (34B) umfasst, die sich von der Basis (34A) erstrecken, und wobei die mehreren vorstehenden Elemente (34B) von einer Seite (30A) der laminierten Anordnung (30) vorstehen.

2. Absorbierender Artikel nach Anspruch 1, wobei die Trägerschicht (32) einen Vliesstoff (36, 38) umfasst.

3. Absorbierender Artikel nach Anspruch 1 oder 2, wobei die Trägerschicht (32) eine thermoplastische Folie umfasst.

4. Absorbierender Artikel nach Anspruch 3, wobei die thermoplastische Folie eine elastische Folie (40) ist.

5. Absorbierender Artikel nach Anspruch 4, wobei die Basis (34A) des Antirutschstreifens (34) und die elastische Folie (40) jeweils eine Breite aufweisen, wobei die Breite (L34A) der Basis (34A) kleiner als die Breite (L40) der elastischen Folie (40) ist, vorzugweise die Breite der Basis größer als oder gleich 10 % der Breite der elastischen Folie und kleiner als oder gleich 60 % der Breite der elastischen Folie ist.

6. Absorbierender Artikel nach Anspruch 4 oder 5, wobei die Trägerschicht (32) einen ersten Vliesstoff (36), einen zweiten Vliesstoff (38) und die elastische Folie (40) umfasst, wobei die elastische Folie (40) zwischen dem ersten und dem zweiten Vliesstoff (36, 38) laminiert ist.

7. Absorbierender Artikel nach einem der Ansprüche 1 bis 6, wobei im Ruhezustand in einem Bereich, der die vorstehenden Elemente (34B) umfasst, der Antirutschstreifen (34) einen statischen Reibungskoeffizienten gemessen gemäß der Norm ASTM D1894 in der Richtung MD und/oder der Richtung CD größer als oder gleich 0,1, vorzugsweise größer als oder gleich 0,5, noch bevorzugter größer als oder gleich 0,8 und kleiner als oder gleich 10, vorzugsweise kleiner als oder gleich 5, noch bevorzugter kleiner als oder gleich 3 aufweist.

8. Absorbierender Artikel nach einem der Ansprüche 1 bis 7, wobei der Antirutschstreifen (34) einen statischen Reibungskoeffizienten gemessen gemäß der Norm ASTM D1894, wenn der Antirutschstreifen (34) auf 15 % des Wertes im Ruhezustand gedehnt wird, der zwischen 50 % und 150 % des statischen Reibungskoeffizienten im Ruhezustand liegt, aufweist.

9. Absorbierender Artikel nach einem der Ansprüche 1 bis 8, wobei im Ruhezustand die mehreren vorstehenden Elemente (34B) eine Dichte der vorstehenden Elemente von mehr als oder gleich 3 vorstehenden Elementen pro cm², vorzugsweise mehr als oder gleich 10 vorstehenden Elementen pro cm² und weniger als oder gleich 400 vorstehenden Elementen pro cm², vorzugsweise weniger als oder gleich 300 vorstehenden Elementen pro cm² aufweisen.

10. Absorbierender Artikel nach einem der Ansprüche 1 bis 9, wobei im Ruhezustand die mehreren vorstehenden Elemente (34B) ein Motiv aufweisen, das eine Wiederholung eines Motivs des Antirutschstreifens (44) umfasst.

11. Absorbierender Artikel nach einem der Ansprüche 1 bis 10, wobei eine Summe der Oberflächen, die auf der Basis (34A) durch die orthogonalen Projektionen der vorstehenden Elemente (34B) auf der Basis (34A) definiert sind, größer als oder gleich 1 % der Gesamtoberfläche der Basis des Motivs des Antirutschstreifens (44), vorzugsweise größer als oder gleich 5 % und kleiner als oder gleich 40 % der Gesamtoberfläche der Basis des Motivs des Antirutschstreifens (44), vorzugsweise kleiner als oder gleich 35 % ist.

12. Absorbierender Artikel nach einem der Ansprüche 1 bis 11, wobei die Basis (34A) eine Dicke (E34A) aufweist, die größer als oder gleich 10 µm, vorzugsweise größer als oder gleich 15 µm und kleiner als oder gleich 200 µm, vorzugweise kleiner als oder gleich 150 µm ist.

13. Absorbierender Artikel nach einem der Ansprüche 1 bis 12, wobei die vorstehenden Elemente (34A) Widerhaken und/oder Stifte und/oder Stäbe sind, wobei jeder Stab einen Kopf beinhaltet, der an einem Ende des Stabs gegenüber der Basis angeordnet ist.

## Claims

1. An absorbent article, such as a disposable diaper, comprising a laminated assembly (30) comprising a support layer (32) and an anti-slip strip (34), the anti-slip strip (34) comprising an elastomeric material, the support layer (32) and the anti-slip strip (34) being laminated together, the anti-slip strip (34) comprising a base (34A) and a plurality of protruding elements (34B) extending from the base (34A) and the plurality of protruding elements (34B) protruding from a face (30A) of the laminated assembly (30).

2. An absorbent article according to claim 1, wherein the support layer (32) comprises a nonwoven web (36, 38).

3. An absorbent article according to claim 1 or 2, wherein the support layer (32) comprises a thermoplastic film.

4. An absorbent article according to claim 3, wherein the thermoplastic film is an elastic film (40).

5. An absorbent article according to claim 4, wherein the base (34A) of the anti-slip strip (34) and the elastic film (40) each have a width, the width (L34A) of the base (34A) being less than the width (L40) of the elastic film (40), preferably the width of the base being greater than or equal to 10% of the width of the elastic film and less than or equal to 60% of the width of the elastic film.

6. An absorbent article according to claim 4 or 5, wherein the support layer (32) comprises a first nonwoven web (36), a second nonwoven web (38) and the elastic film (40), the elastic film (40) being laminated between the first and second nonwoven webs (36, 38).

7. An absorbent article according to any one of claims 1 to 6, wherein, at rest, in a zone comprising the protruding elements (34B), the anti-slip strip (34) has a coefficient of static friction, measured according to standard ASTM D1894, in the direction MD and/or the direction CD, greater than or equal to 0.1, preferably greater than or equal to 0.5, even more preferably greater than or equal to 0.8 and less than or equal to 10, preferably less than or equal to 5, even more preferably less than or equal to 3.

8. An absorbent article according to any one of claims 1 to 7, wherein the anti-slip strip (34) has a coefficient of static friction, measured according to standard ASTM D1894, when the anti-slip strip (34) is stretched to 15% of the rest value, comprised between 50% and 150% of the coefficient of static friction at rest.

9. An absorbent article according to any one of claims 1 to 8, wherein, at rest, the plurality of protruding elements (34B) has a protruding element density greater than or equal to 3 protruding elements per cm², preferably greater than or equal to 10 protruding elements per cm², and less than or equal to 400 protruding elements per cm², preferably less than or equal to 300 protruding elements per cm².

10. An absorbent article according to any one of claims 1 to 9, wherein, at rest, the plurality of protruding elements (34B) have a pattern comprising a repetition of a anti-slip strip pattern (44).

11. An absorbent article according to any one of claims 1 to 10, wherein a sum of the areas defined on the base (34A) by the orthogonal projections of protruding elements (34B) on the base (34A) is greater than or equal to 1% of the total area of the base of the anti-slip strip pattern (44), preferably greater than or equal to 5% and less than or equal to 40% of the total area of the base of the anti-slip strip pattern (44), preferably less than or equal to 35%.

12. An absorbent article according to any one of claims 1 to 11, wherein the base (34A) has a thickness (E34A) greater than or equal to 10 µm, preferably greater than or equal to 15 µm and less than or equal to 200 µm, preferably less than or equal to 150 µm.

13. An absorbent article according to any one of claims 1 to 12, wherein the protruding elements (34A) are pins and/or studs and/or stems, each stem having a head disposed at an end of the stem opposite the base.
